# EUROPEAN PATENT SPECIFICATION

(11) **EP 3 355 887 B1**
(45) Date of publication and mention of the grant of the patent: **28.10.2026**
(21) Application number: 16784139.4
(22) Date of filing: 28.09.2016
(51) Int. Cl.: A61K 31/473, A61P 25/00, A61P 25/16, A61P 25/28, A61P 25/18, A61P 15/00, A61P 15/10, A61K 9/00, A61K 47/12, A61K 47/20

(54) **AQUEOUS COMPOSITION OF APOMORPHINE FOR SUBCUTANEOUS ADMINISTRATION**
WÄSSRIGE ZUSAMMENSETZUNG VON APOMORPHIN ZUR SUBKUTANEN VERABREICHUNG
COMPOSITION AQUEUSE D'APOMORPHINE POUR ADMINISTRATION SOUS-CUTANÉE

(30) Priority: 28.09.2015 EP 15187194
(43) Date of publication of application: 08.08.2018
(73) Proprietor: EVER Neuro Pharma GmbH, 4866 Unterach am Attersee (AT)
(72) Inventor: SCHNAIT, Heinz, 4863 Seewalchen (AT); TÖLGYESI, Zoltán, 5310 Mondsee (AT); SCHMIDT, Sabine, 5020 Salzburg (AT); HARTWIG, Kerstin, 5330 Fuschl am See (AT); MEIER, Dieter Horst, 5310 Mondsee (AT); SELIGER, Christian, 82407 Wielenbach (DE)
(74) Representative: Vossius & Partner Patentanwälte Rechtsanwälte mbB
(86) International application number: PCT/EP2016/073086
(87) International publication number: WO 2017/055337

(56) References cited:
- WO-A1-02/100377
- WO-A1-2013/007381
- WO-A1-2013/183055
- US-A- 5 939 094

## Description

The present invention relates to an aqueous composition comprising apomorphine or a pharmaceutically acceptable salt or solvate thereof, reduced glutathione (GSH) or a pharmaceutically acceptable salt thereof, and ascorbic acid or a pharmaceutically acceptable salt or derivative thereof, wherein the composition has a pH of about 3 to about 7.4. The aqueous composition according to the invention exhibits superior tolerability and an improved side effect profile, particularly a reduced occurrence of skin nodule formation and panniculitis, when administered subcutaneously. The invention further relates to the composition for use as a medicament, particularly for the treatment of Parkinson's disease.

Idiopathic Parkinson's disease is the second most common neurodegenerative disease worldwide. Although no available therapies alter the underlying neurodegenerative process, symptomatic therapies can improve patient quality of life. An estimated 7 to 10 million people are living worldwide with Parkinson's disease (Parkinson's Disease Foundation, Statistics on Parkinson). The average age of onset of Parkinson's disease (PD) is around 62 years. Most PD cases occur sporadically and are of unknown cause.

Clinically, Parkinson's disease is characterized by rest tremor, rigidity, bradykinesia and gait impairment, known as the "cardinal features" of the disease. Additional features include freezing of gait, postural instability, speech difficulty, autonomic disturbances, sensory alterations, mood disorders, sleep dysfunction, cognitive impairment and dementia, all known as non-dopaminergic features because they do not fully respond to dopaminergic therapy (Olanow and Schapira. Ann Neurol. 2013 Sep;74(3):337-47). Pathologically, the hallmark features of PD are degeneration of pigmented mesostriatal dopaminergic neurons linking the substantia nigra (pars compacta) to the neostriatum (caudate nucleus and putamen). Other affected pigmented nuclei may include the locus ceruleus and dorsal motor nucleus of the vagus and and intracytoplasmatic proteinaceous inclusions known as Lewy bodies. These are composed of misfolded and aggregated proteins. Mutations in α-syn promote misfolding of the protein and the formation of oligomers and aggregates thought to be involved in the cell death process (Olanow and Schapira. Ann Neurol. 2013 Sep;74(3):337-47).

Early in the disease course, dopamine deficiency is the predominant neurochemical abnormality. In the progress of the disease, involvement of nondopaminergic brain regions results in levodopa-resistant motor and non-motor symptoms. Consequently, dopamine replacement therapy with levodopa is the gold standard for the initial treatment of Parkinson's disease. Despite the outstanding reputation of levodopa in the early stages of the disease, disabling fluctuations in motor response and dyskinesias constitute the major threat during long-term therapy.

Motor fluctuations in motor performance eventually develop in >50% of patients with Parkinson's disease treated with oral levodopa for >5 years. In addition, many patients also experience other unpleasant "off-period" phenomena, including mood swings, delusions, anxiety and painful dystonia that coincide with their motor state (Cantello R, et al. Neurol Neurosurg Psychiatry. 1986 Oct;49(10):1182-90; Hardie RJ, et al. Brain. 1984 Jun;107(Pt 2):487-506; Nissenbaum H, et al. Psychol Med. 1987 Nov;17(4):899-904; Quinn NP, et al. Lancet. 1986;327(8494):1366-1369). Initially, these response oscillations exhibit a predictable pattern related to the timing of levodopa intake ("wearing-off" phenomenon) and can be managed by shortening the levodopa-dose intervals, an addition of a monoamine oxidase (MAO)-B inhibitor (like selegiline/deprenyl) or dopamine receptor agonists and the administration of controlled-release preparations of levodopa or catechol-O-methyl-transferase inhibitors (e.g., entacapone). However, in the advanced stages of the disease, patients experience complex and unpredictable motor oscillations "on-off" phenomenon, which are refractory to these conventional therapeutic strategies (Marsden CD, et al. Lancet. 1977 Feb 12;1(8007):345-9).

Eventually, the clinical response closely reflects peripheral L-dopa pharmacokinetics, characterized by a plasma half-life of 1-1.5 hours. While the peripheral pharmacokinetics of L-dopa remain unchanged throughout the course of the illness, pre-synaptic nigrostriatal nerve terminals gradually lose their ability to store dopamine. However, evidence exists for a far more complex basis of the development of motor complications that are likely to be related to long-term unphysiological, pulsatile stimulation of the dopamine receptors and involve changes in striatal gene expression and subsequently in altered firing patterns of the basal ganglia.

Motor complications are divided into motor fluctuations and dyskinesia. With advancing PD patient may begin to fluctuate in motor performance, that is to experience a "wearing-off" (end-of-dose) effect because the motor improvement after a dose of levodopa becomes reduced in duration and parkinsonism reappears. A minority of patients may experience diphasic dyskinesia, in which they exhibit dyskinesia at the beginning of turning "on" and/or at the beginning of turning "off", but have different and less severe or absent dyskinesia at the time of peak levodopa effect. Eventually patients may experience rapid and unpredictable fluctuations between "on" and "off" periods known as the "on-off" phenomenon.

The management of motor fluctuations aims to prolong the effect of individual L-dopa doses by adding adjuvant drugs, such as catechol-O-methyl transferase (COMT) and monoamine oxidase B (MAO-B) inhibitors, as well as changing the intervals between intakes and advising patients to avoid taking L-dopa with meals. Also transdermal dopamine agonists are added to the drug regime or their dose is increased. In some patients, attempts to adjust oral and transdermal medication in the presence of disabling fluctuations and dyskinesias fail after months or years. Further options, which include deep-brain stimulation, a pump system that delivers L-dopa to the jejunum via a gastric tube and the dopamine agonist apomorphine, which is delivered subcutaneously either intermittently or continuously, are therapy options for late stage PD patients suffering from motor fluctuations.

Apomorphine is the oldest dopamine agonist used in clinical practice and is indicated for the treatment of motor symptoms associated with late stage Parkinson's disease, specifically for the acute, intermittent treatment of hypomobility, "off" episodes ("end-of-dose wearing off" and unpredictable "on/off" episodes) associated with advanced Parkinson's disease, as adjunct/supplemental therapy to standard levodopa therapy. It was first applied in PD patients in 1951, but interest waned when oral L-dopa was introduced. As the long-term complications associated with L-dopa therapy became recognized, and the antiemetic domperidone (apomorphine leads to severe emesis), which in doses of 10-30 mg tds for 72 hours before apomorphine can prevent most peripheral dopaminergic side effects, became available, apomorphine was investigated further.

Apomorphine is not effective orally due to extensive first-pass metabolism in the liver. The precise mechanism of action of apomorphine as a treatment for Parkinson's disease is unknown, although it is believed to be due to stimulation of post-synaptic D2 receptors within the caudate putamen, a brain structure which supports motor function.

There are currently two distinct methods of administering apomorphine: subcutaneous bolus doses and continuous infusion. When injected subcutaneously, its bioavailability reaches nearly 100% and injections can be effective in rapidly resolving off states in patients with motor fluctuations. When given as a single dose, symptom relief is equivalent to oral L-dopa, with a considerably faster onset (five to 15 minutes) and shorter duration (mean 40 minutes) of effect. Intermittent apomorphine injections may be used to reduce off time in people with PD with severe motor complications. Continuous subcutaneous infusions of apomorphine may be used to reduce "off" time and dyskinesia in people with PD with severe motor complications. Subcutaneous infusions of apomorphine are appropriate for PD people with so many off periods that repeated bolus injections are inappropriate.

Apomorphine, synthesized from morphine by heating with HCI as a chinol derivative, is known to be sensitive for oxidation. Under the influence of oxygen, solutions of apomorphine turn into green color, indicating the formation of oxidation products with "quinone-background" (Neef C, et al. Clin Pharmacokinet. 1999. 37(3):257-71). Electrochemical oxidation experiments have shown that the oxidative apomorphine degradation is pH-dependent. Degradation products increase with increasing pH, leading to a spontaneous autooxidation at neutral pH (Garrido JM, et al. Bioelectrochemistry. 2002. 55(1-2):113-4).

Recently pharmacological autooxidative products of apomorphine were investigated leading to different degradation products in dependency of the pH. At pH < 7 the main degradation product is oxoapomorphine, whereas at pH > 7 apomorphine-paraquinone is the main degradation product (Udvardy A, et al. Journal of molecular structure. 2011. 1002(1):37-44). Acceleration of autooxidation starts with reaching pH 6 and follows reaction kinetic of pseudo-first-order (Garrido JM, et al. J Chem Soc, Perkin Trans 2. 2002. 10:1713-7; Neef C, et al. Clin Pharmacokinet. 1999. 37(3):257-71).

Therefore, apomorphine solutions are commonly protected with sodium metabisulfite as antioxidant and kept in an oxygen reduced environment at a pH between 3 and 4. Using sodium metabisulfite as oxidation protection leads over time to a decrease of pH caused by oxidative degradation of metabisulfite to sulfuric acid. Therefore, buffering of apomorphine solutions should be applied to stabilize the pH over time. For stabilization of apomorphine-containing blood samples pyrosulphate, ascorbic acid or glutathione can be added to the blood sample (Neef C, et al. Clin Pharmacokinet. 1999. 37(3):257-71). Protective effects of the antioxidants cysteine, glutathione and dithiothreitol have also been reported (Maggio R, et al. Neurotox Res. 2000. 1(4):285-97; dos Santos EI-Bachá R, et al. Biochem Pharmacol. 2001. 61(1):73-85).

Water solubility of apomorphine is in pure water at acidic pH 20 mg/ml, whereas in NaCl solution solubility decreases to lower 10 mg/ml. The pKₐ values of the apomorphine-hydrochloride are 7.2 and 8.9, respectively. UV absorption maxima in 0.1 mM HCI solution is at 273 nm and a small shoulder at 305 nm (Muhtadi FJ, et al. Analytical Profiles of Drug Substances. 1991. 20:121-171; also confirmed by own data).

Many routes of administration of apomorphine have been tried in clinical approaches resulting in therapeutic effective application as subcutaneous, sublingual, nasal or rectal administration. Currently only subcutaneous formulations are used in clinical routine (Neef C, et al. Clin Pharmacokinet. 1999. 37(3):257-71).

When administered subcutaneously, apomorphine is known to induce adverse effects at the site of administration, such as skin changes, irritabilities at injection sites and subcutaneous nodules and panniculitis (inflammation of the subcutaneous adipose tissue).

Apomorphine formulations available on the market are stabilized by low pH (3-4) and sodium metabisulfite as antioxidative substance. In some cases sulphites can induce allergic reactions in some patients. In addition sodium metabisulfite tends to react irreversibly with carbon-oxygen double bonds found in aldehydes and ketones. This is evaluated, e.g., for epinephrine, which similar to apomorphine contains two aromatic hydroxyl bound groups in ortho position leading to quinone formation during oxidation (Gupta PK, et al. (eds.). Injectable drug development: techniques to reduce pain and irritation. Taylor and Francis Group. 1999. 409).

Histological data have shown that apomorphine induces melanin-positive pigmentation in the s.c. area (Loewe R, et al. Hautarzt. 2003. 54:58-63). Additional data support the described nodule formation at the injection site as a panniculitis with eosinophile and neutrophile infiltration without increased IgE infiltration (Acland KM, et al. Br J Dermatol. 1998. 138(3):480-2). These irritations lead to a termination of therapy in 70% of the patients receiving apomorphine by subcutaneous infusion within one year.

Nodule formation induced by subcutaneous apomorphine application is one of the most described side effects in injection or infusion therapy with apomorphine. In a meta analysis the incidence of nodule formation was determined with 70% in subcutaneous apomorphine infusion therapy (Deleu D, et al. Drugs Aging. 2004. 21(11):687-709). This side effect is also described in all SmPCs of apomorphine solutions.

However, even if there are descriptions of symptoms, a clear root cause of nodule formations is still missing. For example, Edwards et al. recently noted that "Few studies have been conducted on the formation of apomorphine nodules and consequently, little is known about their aetiology or natural history." (Edwards H, et al. Ultrasound. 2008. 16(3):155-9). Allergic reactions, hygienic reasons, effects induced by the excipients (EDTA or sodium metabisulfite) and dopamine toxicity have been discussed as root cause of nodule formation, without clear evidence for any of these hypotheses (Acland KM, et al. Br J Dermatol. 1998. 138(3):480-2; Boyle A, et al. CNS Drugs. 2015. 29:83-9; Dadban A, et al. Annales de Dermatologie et de Vénéréologie. 2010. 137(11):730-5; Deleu D, et al. Drugs Aging. 2004. 21(11):687-709; Edwards H, et al. Ultrasound. 2008. 16(3):155-9; Ganesaligam J, et al. Movement Disorders. 2011. 26(12):2182; Henriksen T. Neurodegen. Dis. Manage. 2014. 4(3):271-82; Hughes AJ, et al. Movement Disorders. 1993. 8(2):165-70; Loewe R, et al. Hautarzt. 2003. 54:58-63; Martinez-Martin P, et al. Movement Disorder. 2015. 30(14):510-6; Neef C, et al. Clin Pharmacokinet. 1999. 37(3):257-71; Rosei MA, et al. Biochemistry and Molecular Biology International. 1995. 35(6):1253-9).

Several trials were made in the past to explain the neuro-toxic effect of apomorphine on a cellular basis. The results of these investigations described in the following can be of help for understanding the mechanism behind the described side effects of apomorphine-formulations at the subcutaneous injection site. In cytotoxicity studies it could be demonstrated that apomorphine has an anti-proliferative effect and induces apoptosis on the CHO-K1 cell line (Maggio R, et al. Neurotox Res. 2000. 1(4):285-97; Pardini C, et al. Neuropharmacology. 2003. 45(2):182-9). Other studies on rat glioma C6 cells and rat cultured neurons showed that apomorphine promotes the loss of cell membrane integrity, degeneration of cytoplasmic organelles (especially mitochondria), DNA fragmentation and necrosis in vitro most likely through the formation of oxidative degradation products of apomorphine (quinones) (EI-Bachá RS, et al. Neuroscience Letters. 1999. 263:25-8; dos Santos EI-Bachá R, et al. Biochem Pharmacol. 2001. 61(1):73-85). Further it was shown that apomorphine exerts an anti-proliferative effect on several tumor cell lines (Kondo Y, et al. J Pharmacobiodyn. 1990. 13(7):426-31; Schrell UM, et al. J Clin Endocrinol Metab. 1990. 71(6):1669-71).

Also a genotoxic activity of apomorphine was demonstrated in vitro and in vivo and might be related to its ability to intercalate into DNA or to its pro-oxidant effects or generation of superoxide radicals during autoxidation, hence promoting frameshift mutations and inducing oxidative mutagenesis. These mutagenic and clastogenic effects are most likely due to quinone products formed by oxidation of apomorphine (reviewed in: Picada JN, et al. Brazilian journal of medical and biological research. 2005. 38:477-86; Picada JN, et al. Mutat Res. 2003. 539(1-2):29-41; Picada JN, et al. Brain Res Mol Brain Res. 2003. 114(1):80-5) as the more aromatic and planar structure of quinone products favor the intercalation into DNA (Cheng H, et al. Analytical Chemistry. 1979. 51(13):2243-6; Kalyanaraman B. Methods Enzymol. 1990. 186:333-43). It is known that quinones in general are metabolically active intermediates with a toxicological potential leading to several toxic effects in vivo (Garrido JM, et al. J Chem Soc, Perkin Trans 2. 2002. 10:1713-7). Apomorphine was not evidently genotoxic in the in vivo studies performed, however, genotoxic effects of apomorphine and/or its oxidative products as well as its ability to intercalate into DNA can lead to cell death and might be an explanation for the cytotoxic and anti-proliferative effects of apomorphine observed in the studies mentioned above.

Apomorphine can undergo spontaneous autooxidation in neutral and alkaline solutions (Kaul PN. J Pharm Sci. 1961. 50:266-7), which reflects the physiological environment of the subcutaneous tissue and reactive metabolites, such as quinones and reactive oxygen species (ROS) may be produced during this oxidative mechanism. Yet, even in acidic solutions a significant oxidative degradation of apomorphine occurs in the absence of antioxidants, resulting in a green coloration of the solution within a single day. At pH < 7 the main degradation product is oxoapomorphine, whereas at pH > 7 apomorphine-paraquinone is the main degradation product (Udvardy A, et al. Journal of molecular structure. 2011. 1002(1):37-44). Therefore the data from in vitro studies, mentioned above, fit into commonly observed adverse effects of apomorphine at the site of administration, such as skin changes, irritabilities at injection sites and subcutaneous nodules and panniculitis, as oxidation of apomorphine subcutaneously and the generation of oxidative products of apomorphine, such as quinones or semi-quinones, may constitute the mechanisms leading to the loss of cellular integrity and cell death (necrosis) that subsequently trigger the onset of the observed inflammatory processes and nodule formation in the surrounding area of the injection site.

Formulations of apomorphine and therapeutic uses thereof have further been described, e.g., in EP-A-2545905, US 5,939,094, US 6,121,276, US 8,772,309, WO 99/66916, WO 02/100377, WO 2009/019463, WO 2009/056851, WO 2013/007381, and WO 2013/183055.

It is an object of the present invention to provide a novel formulation of apomorphine that can be administered subcutaneously and has improved safety and tolerability and an improved side effect profile. In particular, it is an object of the invention to provide an improved formulation of apomorphine for subcutaneous administration that allows to prevent or reduce the occurrence of inflammatory reactions, nodule formation and panniculitis in the subcutaneous tissue at the site of administration.

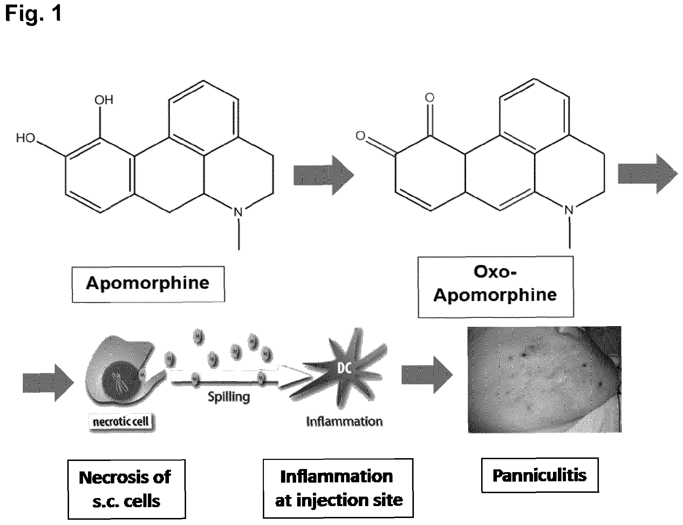

In the context of the present invention, it has surprisingly been found that an aqueous composition comprising apomorphine and a combination of the two antioxidants glutathione and ascorbic acid, having a pH of about 3 to about 7.4, and optionally comprising α-propylene glycol and/or sodium chloride shows superior tolerability, improved stability both under storage conditions and under physiological conditions in the subcutaneous tissue, as well as an improved side effect profile, in particular a considerably reduced occurrence of inflammation and panniculitis at the site of subcutaneous administration. It has further been found that it is oxoapomorphine, an oxidative degradation product of apomorphine which can be formed during storage but is more rapidly formed under physiological conditions in the subcutaneous tissue, that causes panniculitis and other adverse reactions at the site of administration. Oxoapomorphine is toxic to the cells in the subcutaneous tissue, including fibroblasts and adipocytes, and can cause their necrosis, which triggers inflammatory processes that can ultimately lead to panniculitis at the injection site (see Figure 1 and Example 3). The aqueous composition provided in accordance with the present invention, comprising glutathione in combination with ascorbic acid, is highly effective in suppressing these local adverse effects including panniculitis at the site of subcutaneous administration, which is advantageous in terms of tolerability and safety and further facilitates patient acceptance and compliance.

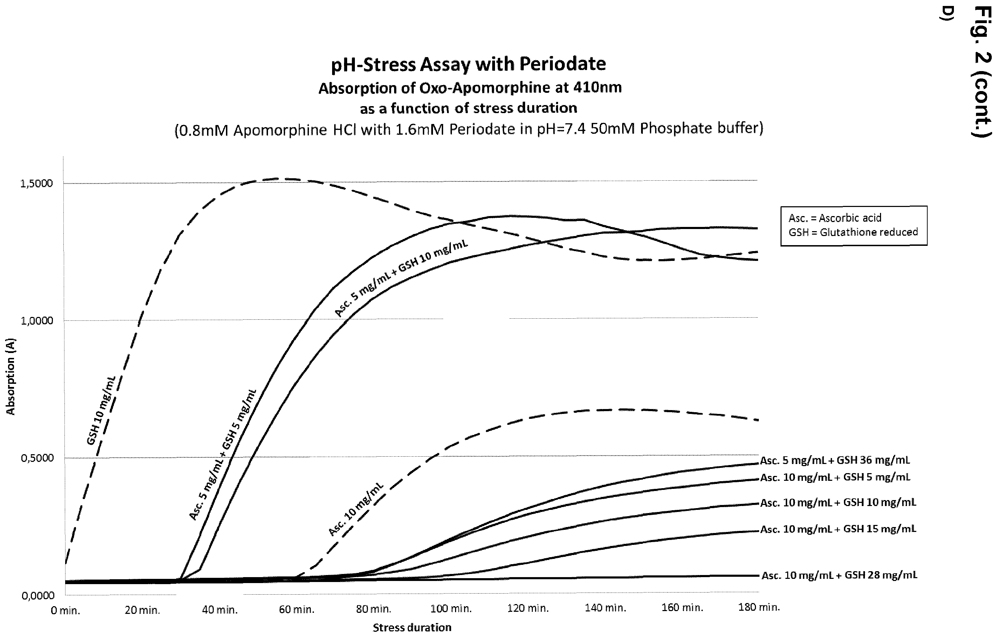

In particular, it has surprisingly been found that the aqueous apomorphine composition according to the present invention, which contains reduced glutathione in combination with ascorbic acid, exhibits a synergistically enhanced stability against oxidative degradation as compared to corresponding apomorphine formulations that contain only reduced glutathione or only ascorbic acid (see Example 1 and Figure 2D). Since the oxidative degradation product oxoapomorphine is considered to be causative for adverse reactions at the site of subcutaneous administration, including panniculitis, the synergistically enhanced stability of the aqueous apomorphine composition of the present invention against oxidative degradation is considered to translate into a synergistically improved reduction of panniculitis and other local adverse effects of subcutaneously administered apomorphine.

The present invention is defined by the independent claims. Any references in the description to methods of treatment refer to the compounds, pharmaceutical compositions and medicaments of the present invention for use in a method for treatment of the human (or animal) body by therapy.

Accordingly, in a first aspect, the invention provides an aqueous composition comprising:
apomorphine or a pharmaceutically acceptable salt or solvate thereof;
reduced glutathione (GSH) or a pharmaceutically acceptable salt thereof; and
ascorbic acid or a pharmaceutically acceptable salt or derivative thereof;
wherein the composition has a pH of about 3 to about 7.4.

In a second aspect, the invention relates to the aqueous composition according to the first aspect for use as a medicament. In accordance with this second aspect, the invention provides a pharmaceutical composition consisting of the aqueous composition according to the first aspect. The invention likewise refers to the aqueous composition according to the first aspect, wherein said composition is a pharmaceutical composition.

In a third aspect, the present invention refers to the aqueous composition according to the first aspect for use in the treatment or prevention of a neurodegenerative disease/disorder. In this third aspect, the invention also relates to the use of the aqueous composition according to the first aspect in the preparation of a medicament for the treatment or prevention of a neurodegenerative disease/disorder. The invention further provides a method of treating a neurodegenerative disease/disorder, the method comprising administering the aqueous composition according to the first aspect to a subject (e.g., a human) in need thereof. In accordance with the third aspect of the invention, the neurodegenerative disease/disorder is preferably selected from Parkinson's disease, Alzheimer's disease, Huntington's disease, neuroleptic malignant syndrome, dystonia, and schizophrenia (e.g., chronic schizophrenia), and is more preferably Parkinson's disease.

The invention is particularly concerned with the treatment or prevention of Parkinson's disease using the aqueous composition according to the first aspect as described and defined herein. Accordingly, in a fourth aspect, the present invention relates to the aqueous composition according to the first aspect for use in the treatment or prevention of Parkinson's disease (e.g., idiopathic Parkinson's disease, acquired Parkinson's disease, or hereditary Parkinson's disease), preferably in a human. In this aspect, the invention further refers to the use of the aqueous composition according to the first aspect in the preparation of a medicament for the treatment or prevention of Parkinson's disease. In the fourth aspect, the invention likewise provides a method of treating Parkinson's disease, the method comprising administering the aqueous composition according to the first aspect to a subject (e.g., a human) in need thereof.

The aqueous composition according to the first aspect of the invention can also be used as a rescue treatment of subjects suffering from Parkinson's disease. In particular, the aqueous composition can be used as an acute treatment of parkinsonian subjects who have been receiving a medication (particularly a chronic medication) different from apomorphine or a salt or solvate thereof and who are suffering from an acute off-period. The aqueous composition can thus be administered on demand when a subject receiving a different treatment of Parkinson's disease (e.g., levodopa) experiences motor fluctuations between regular treatment doses (e.g., between regular doses of levodopa). The aqueous composition may also be administered to subjects who suffer from off-periods of more than about 30 min.

Thus, in a fifth aspect, the present invention relates to the aqueous composition according to the first aspect for use in the treatment or prevention of refractory motor fluctuations/oscillations in Parkinson's disease, off-periods in Parkinson's disease, refractory off-periods in Parkinson's disease, dyskinesia (particularly peak-dose dyskinesia) in Parkinson's disease, or akinesia in Parkinson's disease. In this fifth aspect, the invention further refers to the use of the aqueous composition according to the first aspect in the preparation of a medicament for the treatment or prevention of refractory motor fluctuations/oscillations in Parkinson's disease, off-periods in Parkinson's disease, refractory off-periods in Parkinson's disease, dyskinesia in Parkinson's disease, or akinesia in Parkinson's disease. The invention also relates to a method of treating refractory motor fluctuations/oscillations in Parkinson's disease, off-periods in Parkinson's disease, refractory off-periods in Parkinson's disease, dyskinesia in Parkinson's disease, or akinesia in Parkinson's disease, the method comprising administering the aqueous composition according to the first aspect to a subject (e.g., a human) in need thereof.

In a sixth aspect, the invention provides the aqueous composition according to the first aspect for use in the treatment or prevention of sexual dysfunction or impotence (including male or female sexual dysfunction, particularly male erectile dysfunction), preferably in a human subject. In accordance with this sixth aspect, the invention thus relates, in particular, to the aqueous composition according to the first aspect for use in the treatment or prevention of male erectile dysfunction in a human subject. In this sixth aspect, the invention likewise refers to the use of the aqueous composition according to the first aspect in the preparation of a medicament for the treatment or prevention of sexual dysfunction or impotence, particularly for the treatment or prevention of male erectile dysfunction in a human subject. The invention also provides a method of treating sexual dysfunction or impotence, the method comprising administering the aqueous composition according to the first aspect to a subject (e.g., a human) in need thereof. In particular, the invention provides a method of treating male erectile dysfunction, the method comprising administering the aqueous composition according to the first aspect to a human subject in need thereof.

In a seventh aspect, the invention is directed to the aqueous composition according to the first aspect for use in the treatment or prevention of restless legs syndrome. In accordance with this seventh aspect, the invention also provides the use of the aqueous composition according to the first aspect in the preparation of a medicament for the treatment or prevention of restless legs syndrome. Likewise, the invention provides a method of treating restless legs syndrome, the method comprising administering the aqueous composition according to the first aspect to a subject (e.g., a human) in need thereof.

In an eighth aspect, the present invention relates to the aqueous composition according to the first aspect for use in preventing, reducing or ameliorating panniculitis associated with the subcutaneous administration of apomorphine in the context of the claimed diseases, wherein the composition is to be administered subcutaneously. In this aspect, the invention also relates to the aqueous composition according to the first aspect for use in preventing, reducing or ameliorating the formation of subcutaneous nodules associated with the subcutaneous administration of apomorphine in the context of the claimed diseases, wherein the composition is to be administered subcutaneously, and the invention further relates to the aqueous composition according to the first aspect for use in preventing, reducing or ameliorating inflammation and/or irritation of the skin associated with the subcutaneous administration of apomorphine in the context of the claimed diseases, wherein the composition is to be administered subcutaneously.

In accordance with the eighth aspect, the invention furthermore refers to (i) the use of the aqueous composition according to the first aspect in the preparation of a medicament for preventing, reducing or ameliorating panniculitis associated with the subcutaneous administration of apomorphine in the context of the claimed diseases, wherein the medicament is to be administered subcutaneously, (ii) the use of the aqueous composition according to the first aspect in the preparation of a medicament for preventing, reducing or ameliorating the formation of subcutaneous nodules associated with the subcutaneous administration of apomorphine in the context of the claimed diseases, wherein the medicament is to be administered subcutaneously, and also (iii) the use of the aqueous composition according to the first aspect in the preparation of a medicament for preventing, reducing or ameliorating inflammation and/or irritation of the skin associated with the subcutaneous administration of apomorphine in the context of the claimed diseases, wherein the medicament is to be administered subcutaneously.

In the eighth aspect, the invention likewise refers to (i) a method of preventing, reducing or ameliorating panniculitis associated with the subcutaneous administration of apomorphine in the context of the claimed diseases, the method comprising subcutaneously administering the aqueous composition according to the first aspect to a subject (e.g., a human) in need thereof, (ii) a method of preventing, reducing or ameliorating the formation of subcutaneous nodules associated with the subcutaneous administration of apomorphine in the context of the claimed diseases, the method comprising subcutaneously administering the aqueous composition according to the first aspect to a subject (e.g., a human) in need thereof, and (iii) a method of preventing, reducing or ameliorating inflammation and/or irritation of the skin associated with the subcutaneous administration of apomorphine in the context of the claimed diseases, the method comprising subcutaneously administering the aqueous composition according to the first aspect to a subject (e.g., a human) in need thereof.

The following detailed description applies to all embodiments of the present invention, including all embodiments according to each one of the first, second, third, fourth, fifth, sixth, seventh and eighth aspect as described herein above.

The aqueous composition according to the present invention comprises apomorphine, i.e. (6a*R*)-6-methyl-5,6,6a,7-tetrahydro-4*H*-dibenzo[de,g]quinoline-10,11-diol (also referred to herein as the "free base" form of apomorphine), or a pharmaceutically acceptable salt and/or solvate thereof.

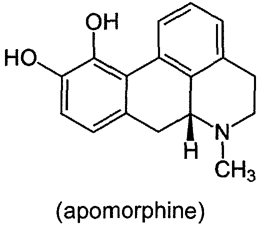

Pharmaceutically acceptable salts of apomorphine may be acid addition salts formed with an inorganic acid such as, e.g., hydrochloric acid, hydrobromic acid, hydroiodic acid, phosphoric acid, sulfuric acid, or perchloric acid, or formed with an organic acid such as, e.g., acetic acid, oxalic acid, maleic acid, malic acid, malonic acid, tartaric acid, citric acid, or succinic acid. Furthermore, pharmaceutically acceptable salts of apomorphine include, without being limited thereto, acetate, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalene-sulfonate, nicotinate, nitrate, oleate, oxalate, palmitate, pamoate, pectinate, perchlorate, persulfate, 3-phenylpropionate, phosphate, picrate, pivalate, propionate, stearate, succinate, sulfate, tartrate, thiocyanate, p-toluene-sulfonate, undecanoate, or valerate salts. A preferred pharmaceutically acceptable salt of apomorphine is the hydrochloride, particularly apomorphine hydrochloride hemihydrate.

The aqueous composition of the invention may comprise apomorphine or a pharmaceutically acceptable salt thereof in any solvated form, including in particular solvates with water. Accordingly, pharmaceutically acceptable solvates of apomorphine or of a pharmaceutically acceptable salt of apomorphine also include hydrates. A preferred pharmaceutically acceptable solvate is apomorphine hydrochloride hemihydrate, i.e. apomorphine • HCI • ½ H₂O (CAS 41372-20-7):

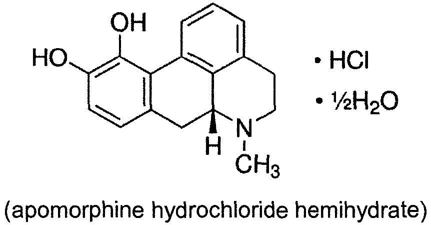

It is preferred that the aqueous composition comprises apomorphine in the form of the hydrochloride salt, particularly in the form of the hydrochloride hemihydrate, i.e., it is particularly preferred that the composition comprises apomorphine hydrochloride hemihydrate. Accordingly, the pharmaceutically acceptable salt or solvate of apomorphine is preferably apomorphine hydrochloride hemihydrate.

Apomorphine and pharmaceutically acceptable salts and solvates thereof are commercially available (e.g., from Sigma-Aldrich Co. LLC, St. Louis, MO, USA), or can be prepared from commercially available forms of apomorphine, or can be prepared as described, e.g., in: Neumeyer JL, et al. J Pharm Sci. 1970. 59(12):1850-1852; Neumeyer JL, et al. J Med Chem. 1973. 16(11):1223-1228; Ram VJ, et al. J Org Chem. 1981. 46(13):2830-2831; or Csutoráas C, et al. Synthetic Communications. 1996. 26(12):2251-2256.

Apomorphine has been described to be useful in the treatment or prevention of Parkinson's disease (e.g., Schwab RS, et al. Trans Am Neurol Assoc. 1951. 56:251-253; Cotzias GC, et al. N Engl J Med. 1970. 282:31-33; Poewe W, et al. Mov Disord. 2000. 15(5):789-794; and Manson AJ, et al. Mov Disord. 2002. 17(6):1235-1241), Alzheimer's disease (e.g., Lashuel HA, et al. J Biol Chem. 2002. 277(45):42881-42890; Steele JW, et al. Ann Neurol. 2011. 69(2):221-225; and Himeno E, et al. Ann Neurol. 2011. 69(2):248-256), Huntington's disease (e.g., Corsini GU, et al. Arch Neurol. 1978. 35(1):27-30; Colosimo C, et al. Clin Neuropharmacol. 1994. 17(3):243-259; and Albanese A, et al. Clin Neuropharmacol. 1995. 18(5):427-434), neuroleptic malignant syndrome (e.g., Colosimo C, et al. Clin Neuropharmacol. 1994. 17(3):243-259; and Wang HC, et al. Mov Disord. 2001. 16(4):765-767), dystonia (e.g., Colosimo C, et al. Clin Neuropharmacol. 1994. 17(3):243-259), schizophrenia (e.g., Smith RC, et al. J Neural Transm. 1977. 40(2):171-176; and Tamminga CA, et al. Science. 1978. 200(4341):567-568), and further neurodegenerative diseases/disorders (e.g., Kyriazis M. J Anti Aging Med. 2003. 6(1):21-28; and Truong JG, et al. Eur J Pharmacol. 2004. 492(2-3):143-147). The use of apomorphine for the treatment or prevention of erectile dysfunction and impotence has also been described in the literature (e.g., Heaton JP, et al. Urology. 1995. 45(2):200-206; O'Sullivan JD, et al. Mov Disord. 1998. 13(3):536-539; Dula E, et al. Urology. 2000. 56(1):130-135; and Rampin O. BJU Int. 2001. 88 Suppl. 3:22-24). The aqueous composition according to the present invention can be used for the treatment or prevention of the above-mentioned disorders as well as any other disorders for which the use of apomorphine has been proposed in the literature.

The aqueous composition preferably comprises apomorphine or a pharmaceutically acceptable salt or solvate thereof (particularly apomorphine hydrochloride hemihydrate) in an amount of about 3 mg/ml to about 20 mg/ml, and more preferably in an amount of about 5 mg/ml to about 10 mg/ml. It is particularly preferred that the aqueous composition comprises apomorphine or a pharmaceutically acceptable salt or solvate thereof (such as apomorphine hydrochloride hemihydrate) in an amount of about 5 mg/ml or in an amount about 10 mg/ml, most preferably in an amount of about 5 mg/ml. It is to be understood that the aforementioned amounts/concentrations are based on the weight of the apomorphine or the pharmaceutically acceptable salt or solvate thereof, i.e., they are not based on the weight of the free base form of apomorphine unless apomorphine is actually used in the free base form (for example, if apomorphine is present in the form of apomorphine hydrochloride hemihydrate, then 5 mg/ml refers to an amount of 5 mg of apomorphine hydrochloride hemihydrate in 1 ml of the aqueous composition). High concentrations of apomorphine or a pharmaceutically acceptable salt or solvate thereof (such as, e.g., 20 mg/ml or more) can be obtained, e.g., if α-propylene glycol or another agent enhancing the solubility of apomorphine is used in the aqueous composition (see Example 2).

An aqueous composition comprising about 5 mg/ml of apomorphine or a pharmaceutically acceptable salt or solvate thereof is advantageous in that it can be directly administered as a "ready-to-use" composition via the subcutaneous route, e.g., by subcutaneous injection. Aqueous compositions comprising higher concentrations of apomorphine or a pharmaceutically acceptable salt or solvate thereof, such as 10 mg/ml, can be used, e.g., for subcutaneous continuous infusion, where the aqueous composition may be diluted to a desired final concentration (e.g., 5 mg/ml) upon administration, or for subcutaneous administration by intermittent bolus injection using an injection pen, where the aqueous composition is prefilled into a pen cartridge (also referred to as a "karpule"). Accordingly, the aqueous composition may be provided, e.g., in a container (such as an injection vial) containing 20 ml of the aqueous composition having a content of apomorphine hydrochloride hemihydrate of 5 mg/ml, or in a pen cartridge containing 3 ml of the aqueous composition having a content of apomorphine hydrochloride hemihydrate of 10 mg/ml.

The aqueous composition according to the invention comprises reduced glutathione or a pharmaceutically acceptable salt thereof. Reduced glutathione (which is also referred to as "GSH") is a tripeptide, i.e. γ-L-glutamyl-L-cysteinylglycine, having a peptide linkage between the γ-carboxyl group of the glutamate residue and the α-amino group of the cysteine residue (IUPAC name: (2S)-2-amino-4-{[(1R)-1-[(carboxymethyl)carbamoyl]-2-sulfanylethyl]carbamoyl}butanoic acid), and functions as an antioxidant.

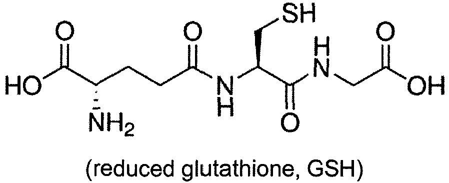

The oxidized form of glutathione (which is also referred to as "GSSG") is glutathione disulfide (IUPAC name: (2S)-2-amino-5-[[(2R)-3-[(2R)-2-[[(4S)-4-amino-5-hydroxy-5-oxopentanoyl]amino]-3-(carboxymethylamino)-3-oxopropyl]disulfanyl-1-(carboxymethylamino)-1-oxopropan-2-yl]amino]-5-oxopentanoic acid).

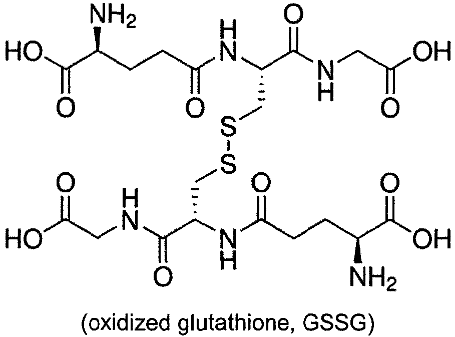

Reduced glutathione (GSH) is commercially available in pharmaceutical quality (e.g., from Sigma-Aldrich Co. LLC, St. Louis, MO, USA) or can be prepared from commercially available precursors.

Pharmaceutically acceptable salts of reduced glutathione (GSH) may be formed, e.g., by protonation of an atom carrying an electron lone pair which is susceptible to protonation, such as an amino group, with an inorganic or organic acid, or as a salt of an acid group (such as a carboxylic acid group) with a physiologically acceptable cation. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts. Exemplary acid addition salts comprise, for example: mineral acid salts such as hydrochloride, hydrobromide, hydroiodide, sulfate salts (such as, e.g., sulfate or hydrogensulfate salts), nitrate salts, phosphate salts (such as, e.g., phosphate, hydrogenphosphate, or dihydrogenphosphate salts), carbonate salts, hydrogencarbonate salts, perchlorate salts, borate salts, or thiocyanate salts; organic acid salts such as acetate, propionate, butyrate, pentanoate, hexanoate, heptanoate, octanoate, cyclopentanepropionate, decanoate, undecanoate, oleate, stearate, lactate, maleate, oxalate, fumarate, tartrate, malate, citrate, succinate, adipate, gluconate, glycolate, nicotinate, benzoate, salicylate, ascorbate, pamoate (embonate), camphorate, glucoheptanoate, or pivalate salts; sulfonate salts such as methanesulfonate (mesylate), ethanesulfonate (esylate), 2-hydroxyethanesulfonate (isethionate), benzenesulfonate (besylate), p-toluenesulfonate (tosylate), 2-naphthalenesulfonate (napsylate), 3-phenylsulfonate, or camphorsulfonate salts; glycerophosphate salts; and acidic amino acid salts such as aspartate or glutamate salts. Preferred examples of pharmaceutically acceptable salts of reduced glutathione (GSH) are alkali metal salts. A particularly preferred pharmaceutically acceptable salt of reduced glutathione (GSH) is the sodium salt.

It is preferred that the aqueous composition comprises reduced glutathione (GSH) or a pharmaceutically acceptable salt thereof in an amount of about 1 mg/ml to about 50 mg/ml, more preferably in an amount of about 1 mg/ml to about 20 mg/ml, even more preferably in an amount of about 2 mg/ml to about 10 mg/ml, and still more preferably in an amount of about 5 mg/ml.

The aqueous composition further comprises ascorbic acid or a pharmaceutically acceptable salt or derivative thereof. Preferably, the aqueous composition comprises ascorbic acid or a pharmaceutically acceptable salt thereof. Ascorbic acid (or a pharmaceutically acceptable salt thereof) may be present in the L-form or in the D-form, and is preferably present in the L-form (IUPAC name: (5R)-[(1S)-1,2-dihydroxyethyl]-3,4-dihydroxyfuran-2(5H)-one). It functions as an antioxidant and can be oxidized to form dehydroascorbic acid. Ascorbic acid is commercially available in pharmaceutical quality (e.g., from Sigma-Aldrich Co. LLC, St. Louis, MO, USA).

Pharmaceutically acceptable salts of ascorbic acid may be formed, e.g., as a salt of the ascorbate anion with a physiologically acceptable cation. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts. Preferred pharmaceutically acceptable salts of ascorbic acid include, in particular, sodium ascorbate, potassium ascorbate, or calcium ascorbate. More preferably, the pharmaceutically acceptable salt of ascorbic acid is sodium ascorbate or calcium ascorbate.

Pharmaceutically acceptable derivatives of ascorbic acid include, in particular, pharmaceutically acceptable esters of ascorbic acid, such as esters formed from ascorbic acid and one, two, three or four acids (i.e., one, two, three or four of the hydroxy groups of ascorbic acid may each be esterified with the acid group (or hydroxyacyl group) of an acid) as well as pharmaceutically acceptable salts thereof. The one to four acids from which such esters of ascorbic acid can be formed may, e.g., be selected from C₈₋₂₂ carboxylic acids (such as caprylic acid, capric acid, lauric acid, myristic acid, myristoleic acid, palmitic acid, palmitoleic acid, stearic acid, oleic acid, arachidic acid, or behenic acid) and phosphoric acid. Thus, pharmaceutically acceptable esters of ascorbic acid include, for example, ascorbic acid which is esterified with one C₈₋₂₂ carboxylic acid (e.g., any of the specific examples mentioned above) at any one of its hydroxy groups (e.g., at its 2- hydroxy group or its 6- hydroxy group), ascorbic acid which is esterified with two C₈₋₂₂ carboxylic acids (e.g., any of the specific examples mentioned above) at any two of its hydroxy groups (e.g., at both its 2-hydroxy and its 6-hydroxy group), ascorbic acid which is esterified with four C₈₋₂₂ carboxylic acids (e.g., any of the specific examples mentioned above) at all four of its hydroxy groups, or ascorbic acid which is esterified with one phosphoric acid at any one of its hydroxy groups (e.g., at its 4-hydroxy group). Pharmaceutically acceptable salts of any such esters are also encompassed within the pharmaceutically acceptable derivatives of ascorbic acid. Moreover, pharmaceutically acceptable derivatives of ascorbic acid also include conjugates of ascorbic acid with a sugar (e.g., a monosaccharide, such as glucose) wherein, e.g., any one of the hydroxy groups of ascorbic acid may be condensed with a hydroxy group of the sugar to form an ether linkage, as well as pharmaceutically acceptable salts of such conjugates. Preferred examples of pharmaceutically acceptable derivatives of ascorbic acid include ascorbyl-2-glucoside, ascorbyl-6-octanoate, ascorbyl-6-palmitate, ascorbyl-6-stearate, ascorbyl-2,6-dipalmitate, ascorbyl phosphate (e.g., ascorbyl-4-phosphate), ascorbyl tetraisopalmitate tetrahexyldecyl ascorbate, chitosan ascorbate, as well as pharmaceutically acceptable salts of any one of these derivatives (such as, e.g., sodium ascorbyl phosphate or magnesium ascorbyl phosphate).

It is preferred that the aqueous composition comprises ascorbic acid or a pharmaceutically acceptable salt or derivative thereof in an amount of about 2 mg/ml to about 50 mg/ml, more preferably in an amount of about 5 mg/ml to about 20 mg/ml, even more preferably in an amount of about 8 mg/ml to about 15 mg/ml, and yet even more preferably in an amount of about 10 mg/ml.

The aqueous composition has a pH of about 3 to about 7.4 (such as, e.g., a pH of about 3.0, about 3.7, about 4.0, about 4.5, about 5.0, about 5.5, about 6.0, about 6.5, about 7.0, or about 7.4). It preferably has a pH of about 4 to about 7, more preferably a pH of about 5 to about 6 (e.g., about 5.0, about 5.5, or about 6.0), and even more preferably a pH of about 5.5.

The desired pH may be controlled by using a suitable buffer and, if necessary, adjusting the pH by adding an aqueous solution of HCI or of NaOH. The buffer is not particularly limited and may be selected, e.g., from malate buffer, formate buffer, succinate buffer, citrate buffer, acetate buffer, pyridine buffer, MES buffer, tartrate buffer, oxalate buffer, ascorbate buffer, cacodylate buffer, dimethylglutarate buffer, carbonate buffer, Bis-Tris buffer, ADA buffer, pyrophosphate buffer, EDPS buffer, Bis-Tris propane buffer, PIPES buffer, ACES buffer, MOPSO buffer, imidazole buffer, histidine buffer, BES buffer, MOPS buffer, phosphate buffer, EMTA buffer, TES buffer, HEPES buffer, and DIPSO buffer (as described, e.g., in Stoll VS, et al. Buffers: principles and practice. Methods Enzymol. 1990. 182:24-38; or in AppliChem. Biological Buffers. 2008). It will be understood that a suitable buffer can be selected depending on the pKₐ value of the buffer substance and the desired pH of the aqueous composition.

The ascorbic acid or the pharmaceutically acceptable salt or derivative thereof which is comprised in the aqueous composition according to the invention may not only function as an antioxidant but may also be used as a buffer. If ascorbic acid or a pharmaceutically acceptable salt or derivative thereof is used as a buffer (e.g., if the aqueous composition comprises an ascorbic acid/ascorbate buffer), it is preferred that the aqueous composition has a pH of about 3 to about 6 (e.g., a pH of about 3.1 to about 5.9), more preferably a pH of about 4 to about 5.8, and even more preferably a pH of about 5.5. Accordingly, the composition of the first aspect of the invention may be an aqueous composition comprising: apomorphine or a pharmaceutically acceptable salt or solvate thereof; reduced glutathione (GSH) or a pharmaceutically acceptable salt thereof; and an ascorbic acid/ascorbate buffer; wherein the composition has a pH of about 3 to about 6 (preferably of about 4 to about 5.5, and more preferably of about 5.5).

The aqueous composition according to the invention preferably further comprises α-propylene glycol (i.e., propane-1,2-diol) and/or sodium chloride; more preferably, it comprises α-propylene glycol. These substances can be used as isotonizing agents for rendering the aqueous composition isotonic with human blood plasma. The aqueous composition may comprise α-propylene glycol in an amount of, e.g., about 1 mg/ml to about 25 mg/ml (or about 5 mg/ml to about 15 mg/ml), but if α-propylene glycol is used as an isotonizing agent, the preferred amount of α-propylene glycol will depend on the pH of the aqueous composition (e.g., an aqueous composition according to the invention having a pH of about 5 may preferably contain α-propylene glycol in an amount of about 5 mg/ml to about 10 mg/ml). The aqueous composition may comprise sodium chloride in an amount of, e.g., about 3 mg/ml to about 8 mg/ml, but if sodium chloride is used as an isotonizing agent, the preferred amount of sodium chloride will depend on the pH of the aqueous composition (e.g., an aqueous composition according to the invention having a pH of about 5 may preferably contain sodium chloride in an amount of about 4.5 mg/ml).

It is preferred that the aqueous composition is isotonic with respect to human blood plasma. In particular, it is preferred that the aqueous composition has an osmolality of about 280 mOsm/kg to about 305 mOsm/kg, more preferably an osmolality of about 290 mOsm/kg to about 300 mOsm/kg, and even more preferably an osmolality of about 296 mOsm/kg. Moreover, the aqueous composition is preferably rendered isotonic (e.g., to any of the aforementioned osmolality ranges or values) using α-propylene glycol and/or sodium chloride, more preferably using α-propylene glycol.

The use of α-propylene glycol is also advantageous because it enhances the solubility of apomorphine (or a pharmaceutically acceptable salt/solvate thereof), as demonstrated in Example 2, and thus allows higher concentrations of apomorphine to be included in the aqueous composition and further allows apomorphine to be absorbed more rapidly after subcutaneous administration. Moreover, α-propylene glycol does not only enhance the solubility of apomorphine but also enhances the solubility of its toxic oxidative degradation product oxoapomorphine which would otherwise readily precipitate in an aqueous environment. The enhanced solubility of oxoapomorphine formed from the apomorphine contained in the aqueous composition accelerates the moving away of oxoapomorphine from the site of subcutaneous administration and thereby reduces or prevents the formation of subcutaneous nodules or panniculitis at the injection site. The aqueous composition comprising α-propylene glycol thus has a particularly improved safety and tolerability and a particularly advantageous side effect profile.

The aqueous composition according to the invention comprises water, preferably at least about 60% (v/v) water, more preferably at least about 70% (v/v) water, even more preferably at least about 80% (v/v) water, even more preferably at least about 90% (v/v) water, and yet even more preferably at least about 95% (v/v) water, with respect to the total volume of the aqueous composition. The water in the aqueous composition is preferably water for injection (e.g., as defined in the European Pharmacopoeia (Ph. Eur.), 8th Edition as of July 1, 2015, including supplement 8.6). Water for injection (WFI) can be prepared using techniques known in the art, e.g., by distillation or by membrane technologies (such as reverse osmosis or ultrafiltration), as described, e.g., in Felton LA (ed.), Remington: Essentials of Pharmaceutics, Pharmaceutical Press, 2013.

The aqueous composition may be, e.g., an aqueous solution or an oil-in-water emulsion. In this regard, it is preferred that the aqueous composition has an oil content of less than about 5% (v/v), more preferably of less than about 3% (v/v), even more preferably of less than about 2% (v/v), even more preferably of less than about 1% (v/v), even more preferably of less than about 0.5% (v/v), and yet even more preferably it does not contain any oil. Accordingly, it is preferred that the aqueous composition is an aqueous solution.

Furthermore, it is preferred that the aqueous composition has a total content of lipophilic substances of less than about 5% (v/v), more preferably of less than about 3% (v/v), even more preferably of less than about 2% (v/v), even more preferably of less than about 1% (v/v), even more preferably of less than about 0.5% (v/v), and yet even more preferably it does not contain any lipophilic substances.

The aqueous composition according to the present invention can be formulated by techniques known to the person skilled in the art, such as the techniques published in "Remington: The Science and Practice of Pharmacy", Pharmaceutical Press, 22nd edition. The aqueous composition may optionally further comprise one or more pharmaceutically acceptable excipients, such as solvents and cosolvents, surfactants, complexants, chelating agents, buffering agents, tonicity agents, antioxidants, preservatives, bulking agents, stabilizers, and/or solubility enhancers.

For example, the aqueous composition may comprise one or more solubility enhancers, such as, e.g., poly(ethylene glycol), including poly(ethylene glycol) having a molecular weight in the range of about 200 to about 5,000 Da (e.g., PEG 300), ethylene glycol, glycerol, a non-ionic surfactant, tyloxapol, polysorbate 80, macrogol-15-hydroxystearate (e.g., Kolliphor^{®} HS 15, CAS 70142-34-6), a phospholipid, lecithin, dimyristoyl phosphatidylcholine, dipalmitoyl phosphatidylcholine, distearoyl phosphatidylcholine, a cyclodextrin, α-cyclodextrin, β-cyclodextrin, γ-cyclodextrin, hydroxyethyl-β-cyclodextrin, hydroxypropyl-β-cyclodextrin, hydroxyethyl-γ-cyclodextrin, hydroxypropyl-γ-cyclodextrin, dihydroxypropyl-β-cyclodextrin, sulfobutylether-β-cyclodextrin, sulfobutylether-γ-cyclodextrin, glucosyl-α-cyclodextrin, glucosyl-β-cyclodextrin, diglucosyl-β-cyclodextrin, maltosyl-α-cyclodextrin, maltosyl-β-cyclodextrin, maltosyl-γ-cyclodextrin, maltotriosyl-β-cyclodextrin, maltotriosyl-γ-cyclodextrin, dimaltosyl-β-cyclodextrin, methyl-β-cyclodextrin, a carboxyalkyl thioether, hydroxypropyl methylcellulose, hydroxypropylcellulose, polyvinylpyrrolidone, a vinyl acetate copolymer, vinyl pyrrolidone, sodium lauryl sulfate, dioctyl sodium sulfosuccinate, or any combination thereof.

It is preferred that the aqueous composition according to the invention comprises one or more preservatives, particularly one or more antimicrobial preservatives. Suitable antimicrobial preservatives are known in the art and include, for example, benzyl alcohol, chlorobutanol, 2-ethoxyethanol, m-cresol, chlorocresol (e.g., 2-chloro-3-methyl-phenol or 4-chloro-3-methylphenol), benzalkonium chloride, benzethonium chloride, benzoic acid (or a pharmaceutically acceptable salt thereof), sorbic acid (or a pharmaceutically acceptable salt thereof), chlorhexidine and/or thimerosal. A preferred antimicrobial preservative is benzyl alcohol.

The aqueous composition can be formulated as a dosage form for parenteral administration (such as subcutaneous, intramuscular, or intradermal administration) or for nasal administration, and is preferably formulated as a dosage form for parenteral administration, more preferably for subcutaneous administration. Dosage forms for parenteral administration include solutions, emulsions, suspensions, dispersions, and powders and granules for reconstitution. Solutions and emulsions are preferred dosage forms for parenteral administration. The preparation of suitable parenteral formulations under sterile conditions is readily accomplished by standard pharmaceutical techniques well known to those skilled in the art.

The aqueous composition may be administered to a subject by any convenient route of administration, and is preferably administered parenterally (e.g., using injection techniques or infusion techniques, and including, e.g., subcutaneously, subcuticularly, intramuscularly, or intradermally). Most preferably, however, the aqueous composition is to be administered subcutaneously, e.g., by subcutaneous injection or by subcutaneous infusion. The route of administration in each of the second to seventh aspects is thus preferably by subcutaneous administration, particularly by subcutaneous injection or by subcutaneous infusion.

The aqueous composition according to the invention should preferably be free or at least substantially free of divalent metal cations, particularly since such cations may catalyze the degradation of apomorphine. Divalent metal cations can be removed using methods known in the art (e.g., by pre-treating the various components with suitable cation exchange chromatography systems).

Typically, a physician will determine the actual dosage which will be most suitable for an individual subject. The specific dose level and frequency of dosage for any particular individual subject may be varied and will depend upon a variety of factors including the activity of the specific form of apomorphine (e.g., the specific salt or solvate) employed, the metabolic stability and length of action of this specific form of apomorphine, the age, body weight, general health, sex, diet, mode and time of administration, rate of excretion, drug combination, the severity of the particular condition, and the individual subject undergoing therapy. It is to be understood that the aqueous composition according to the invention is to be administered to a subject in a therapeutically effective amount (i.e., a therapeutically effective amount with respect to the apomorphine or the pharmaceutically acceptable salt or solvate comprised in the aqueous composition).

For example, the aqueous composition according to the invention can be administered to a human subject (preferably a human subject aged 18 or older) by subcutaneous intermittent bolus injection in a dose of about 1 mg to about 10 mg of the active ingredient (i.e., apomorphine or the pharmaceutically acceptable salt or solvate thereof which is comprised in the aqueous composition). The corresponding unit dose may, e.g., be administered subcutaneously 1-12 times per day (preferably 1-10 times per day), up to a maximum daily dose of, e.g., about 100 mg (preferably up to a maximum daily dose of about 50 mg, and more preferably up to a maximum daily dose of about 30 mg). Alternatively, the aqueous composition can also be administered to a human subject (preferably a human subject aged 18 or older) by subcutaneous continuous infusion (e.g., using a minipump and/or a syringe driver) at an infusion rate of about 1 mg to about 4 mg of the active ingredient (i.e., apomorphine or the pharmaceutically acceptable salt or solvate thereof, which is preferably comprised in the aqueous composition in a concentration of about 5 mg/ml) per hour, or at an infusion rate of about 0.015 mg/kg/hour to about 0.06 mg/kg/hour of the active ingredient (i.e., apomorphine or the pharmaceutically acceptable salt or solvate thereof, which is preferably comprised in the aqueous composition in a concentration of about 5 mg/ml). It will be appreciated that it may be necessary to make routine variations to the dosage depending on the age and weight of the patient/subject as well as the severity of the condition to be treated. The precise dose and also the route of administration will ultimately be at the discretion of the attendant physician.

The aqueous composition according to the invention, comprising apomorphine or a pharmaceutically acceptable salt or solvate thereof as active agent, may be administered in the context of a monotherapy or in combination with one or more further pharmaceutically active agents. If the aqueous composition of the invention is used in combination with a further pharmaceutically active agent which is active against the same disease/disorder, a lower dose of each agent may be used. The combination of the aqueous composition according to the present invention with one or more further pharmaceutically active agents may comprise the simultaneous/concomitant administration of the further pharmaceutically active agents with the aqueous composition according to the invention. However, sequential/separate administration is also envisaged. When administration is sequential, either the aqueous composition of the invention or the one or more further pharmaceutically active agents may be administered first. When administration is simultaneous, the one or more further pharmaceutically active agents may be included in the aqueous composition of the invention or may be administered in one or more different (separate) pharmaceutical compositions.

For the treatment or prevention of Parkinson's disease, the aqueous composition of apomorphine (or a pharmaceutically acceptable salt/solvate thereof) according to the present invention can be administered in combination with one or more further antiparkinson agents which may, for example, be selected from etilevodopa, droxidopa, levodopa, melevodopa, aplindore, bromocriptine, cabergoline, ciladopa, dihydroergocryptine, lisuride, pardoprunox, pergolide, piribedil, pramipexole, ropinirole, rotigotine, ladostigil, lazabemide, mofegiline, pargyline, rasagiline, selegiline, entacapone, nitecapone, tolcapone, benserazide, carbidopa, methyldopa, benzatropine, biperiden, bornaprine, chlorphenoxamine, cycrimine, dexetimide, dimenhydrinate, diphenhydramine, etanautine, etybenzatropine, mazaticol, metixene, orphenadrine, phenglutarimide, piroheptine, procyclidine, profenamine, trihexyphenidyl, tropatepine, amantadine, budipine, memantine, methylxanthines, rimantadine, UWA-101, and pharmaceutically acceptable salts and solvates of any of these agents. Preferred antiparkinson agents are levodopa, carbidopa, and biperiden. A particularly preferred antiparkinson agent is levodopa.

Accordingly, the present invention relates to the aqueous composition of the first aspect, as described and defined herein, for use in the treatment or prevention of Parkinson's disease (or for use in the treatment or prevention of refractory motor fluctuations/oscillations in Parkinson's disease, off-periods in Parkinson's disease, refractory off-periods in Parkinson's disease, dyskinesia in Parkinson's disease, and/or akinesia in Parkinson's disease), wherein said aqueous composition is to be administered subcutaneously, and wherein said aqueous composition is to be administered in combination with one or more further antiparkinson agents (e.g., one or more of the specific antiparkinson agents described above). The combined administration of the aqueous composition with one or more further antiparkinson agents may be effected, e.g., by simultaneous/concomitant administration or by sequential/separate administration. The one or more further antiparkinson agents are not necessarily administered subcutaneously but may rather be administered by any convenient route of administration.

Since apomorphine can cause short-term nausea, particularly in the beginning of the treatment, the aqueous composition according to the invention is preferably administered in combination with an anti-emetic agent. Such a combination treatment may, for example, be administered for a period of at least two weeks before the administration of the anti-emetic agent may be terminated while the administration of the aqueous composition of apomorphine is continued. The anti-emetic agent may, for example, be selected from alizapride, alosetron, aprepitant, atropine, azasetron, bemesetron, benzquinamine, bromopride, buclizine, casopitant, cerium oxalate, chlorpromazine, cilansetron, clebopride, clozapine, cyclizine, cyproheptadine, dazopride, dexamethasone, dimenhydrinate, diphenhydramine, diphenidol, dolasetron, domperidone, dronabinol, ezlopitant, fosaprepitant, granisetron, haloperidol, hydroxyzine, hyoscyamine, itopride, lerisetron, lorazepam, maropitant, meclozine, metoclopramide, metopimazine, mianserin, midazolam, mirtazapine, nabilone, nonabine, olanzapine, ondansetron, oxypendyl, palonosetron, pipamazine, prochlorperazine, promethazine, propofol, quetiapine, ramosetron, ricasetron, risperidone, scopolamine, tetrahydrocannabinol, thiethylperazine, trimethobenzamide, tropisetron, vestipitant, zatosetron, ziprasidone, and pharmaceutically acceptable salts (e.g., a hydrochloride) and solvates of any of these agents. A particularly preferred anti-emetic agent is domperidone.

Accordingly, the present invention relates to an aqueous composition, as described and defined herein, wherein said aqueous composition is to be administered subcutaneously, and wherein said aqueous composition is to be administered in combination with an anti-emetic agent (e.g., any one of the anti-emetic agents listed above, preferably domperidone). The combined administration of the aqueous composition with an anti-emetic agent may be effected, e.g., by simultaneous/concomitant administration of the anti-emetic agent with the aqueous composition or, alternatively, by sequential/separate administration. When administration is sequential, either the aqueous composition of the invention or the anti-emetic agent may be administered first. When administration is simultaneous, the anti-emetic agent may be included in the aqueous composition or may be administered in a different (separate) pharmaceutical composition. The anti-emetic agent, if provided in a separate pharmaceutical composition, does not need to be administered subcutaneously but may rather be administered by any convenient route of administration.

The invention also relates to the combined administration of the aqueous composition with an anti-emetic agent (as described above) and with one or more further antiparkinson agents (as described above).

The subject or patient to be treated in accordance with the present invention may be an animal (e.g., a non-human animal), a vertebrate animal, a mammal, a rodent (e.g., a guinea pig, a hamster, a rat, or a mouse), a canine (e.g., a dog), a feline (e.g., a cat), a porcine (e.g., a pig), an equine (e.g., a horse), a primate or a simian (e.g., a monkey or an ape, such as a marmoset, a baboon, a gorilla, a chimpanzee, an orangutan, or a gibbon), or a human. In accordance with the present invention, it is envisaged that animals are to be treated which are economically, agronomically or scientifically important. Scientifically important organisms include, but are not limited to, mice, rats, and rabbits. Lower organisms such as, e.g., fruit flies like *Drosophila melagonaster* and nematodes like *Caenorhabditis elegans* may also be used in scientific approaches. Non-limiting examples of agronomically important animals are sheep, cattle and pigs, while, for example, cats and dogs may be considered as economically important animals. Preferably, the subject/patient is a mammal. More preferably, the subject/patient is a human or a non-human mammal (such as, e.g., a guinea pig, a hamster, a rat, a mouse, a rabbit, a dog, a cat, a horse, a monkey, an ape, a marmoset, a baboon, a gorilla, a chimpanzee, an orangutan, a gibbon, a sheep, cattle, or a pig). Most preferably, the subject/patient is a human.

The term "treatment" of a disorder or disease as used herein is well known in the art. "Treatment" of a disorder or disease implies that a disorder or disease is suspected or has been diagnosed in a patient/subject. A patient/subject suspected of suffering from a disorder or disease typically shows specific clinical and/or pathological symptoms which a skilled person can easily attribute to a specific pathological condition (i.e., diagnose a disorder or disease).

The "treatment" of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above). The treatment of a disorder or disease may, *inter alia,* comprise curative treatment (preferably leading to a complete response and eventually to healing of the disorder or disease) and palliative treatment (including symptomatic relief).

The term "prevention" of a disorder or disease as used herein is also well known in the art. For example, a patient/subject suspected of being prone to suffer from a disorder or disease may particularly benefit from a prevention of the disorder or disease. The subject/patient may have a susceptibility or predisposition for a disorder or disease, including but not limited to hereditary predisposition. Such a predisposition can be determined by standard methods or assays, using, e.g., genetic markers or phenotypic indicators. It is to be understood that a disorder or disease to be prevented in accordance with the present invention has not been diagnosed or cannot be diagnosed in the patient/subject (for example, the patient/subject does not show any clinical or pathological symptoms). Thus, the term "prevention" comprises the use of a compound of the present invention before any clinical and/or pathological symptoms are diagnosed or determined or can be diagnosed or determined by the attending physician.

The term "propylene glycol" refers to both α-propylene glycol (i.e., propane-1,2-diol) and β-propylene glycol (i.e., propane-1,3-diol).

The term "α-propylene glycol" refers to propane-1,2-diol. The propane-1,2-diol may be (R)-propane-1,2-diol, (S)-propane-1,2-diol, or any racemic mixture thereof.

As used herein, the term "carboxylic acid" refers to a compound R-COOH, wherein R is a hydrocarbon group (i.e., a group consisting of carbon atoms and hydrogen atoms), particularly a C₇₋₂₁ hydrocarbon group. A "C₈₋₂₂ carboxylic acid" denotes a carboxylic acid having a total of 8 to 22 carbon atoms (including the carbon atom of the COOH group). Unless defined otherwise, the term "C₈₋₂₂ carboxylic acid" preferably refers to a compound R-COOH, wherein R is a C₇₋₂₁ alkyl group or a C₇₋₂₁ alkenyl group. Exemplary preferred C₈₋₂₂ carboxylic acids are caprylic acid, capric acid, lauric acid, myristic acid, myristoleic acid, palmitic acid, palmitoleic acid, stearic acid, oleic acid, arachidic acid, or behenic acid.

The term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. A "C₇₋₂₁ alkyl" denotes an alkyl group having 7 to 21 carbon atoms.

The term "alkenyl" refers to a monovalent unsaturated acyclic hydrocarbon group which may be linear or branched and comprises one or more (e.g., one or two) carbon-to-carbon double bonds while it does not comprise any carbon-to-carbon triple bond. The term "C₇₋₂₁ alkenyl" denotes an alkenyl group having 7 to 21 carbon atoms.

The term "about" preferably refers to ±10% of the indicated numerical value, more preferably to ±5% of the indicated numerical value, and in particular to the exact numerical value indicated. For example, the expression "about 100" preferably refers to 100 ±10% (i.e., 90 to 110), more preferably to 100 ±5% (i.e., 95 to 105), and even more preferably to the specific value of 100. If the term "about" is used in connection with the endpoints of a range, it preferably refers to the range from the lower endpoint -10% of its indicated numerical value to the upper endpoint +10% of its indicated numerical value, more preferably to the range from of the lower endpoint -5% to the upper endpoint +5%, and even more preferably to the range defined by the exact numerical values of the lower endpoint and the upper endpoint. Thus, the expression "about 10 to about 20" preferably refers to the range of 9 to 22, more preferably to the range of 9.5 to 21, and even more preferably to the range of 10 to 20. If the term "about" is used in connection with the endpoint of an open-ended range, it preferably refers to the corresponding range starting from the lower endpoint -10% or from the upper endpoint +10%, more preferably to the range starting from the lower endpoint -5% or from the upper endpoint +5%, and even more preferably to the open-ended range defined by the exact numerical value of the corresponding endpoint. For example, the expression "at least about 10%" preferably refers to at least 9%, more preferably to at least 9.5%, and even more preferably to at least 10%.

The terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, if a component of a composition is indicated to be "optional", the invention specifically relates to both possibilities, i.e., that the corresponding component is present (contained in the composition) or that the corresponding component is absent from the composition.

The term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

Any parameters referred to herein (including, e.g., any amounts/concentrations indicated in "mg/ml" or in "% (v/v)", and any pH values) are preferably to be determined at standard ambient temperature and pressure conditions, particularly at a temperature of 25°C (298.15 K) and at an absolute pressure of 1 atm (101.325 kPa).

It is to be understood that the present invention specifically relates to each and every combination of features and embodiments described herein, including any combination of general and/or preferred features/embodiments.

In this specification, a number of documents including patent applications and scientific literature are cited.

The invention is also described by the following illustrative figures. The appended figures show:
**Figure 1****:** Oxoapomorphine formed from the oxidative degradation of subcutaneously administered apomorphine causes the necrosis of subcutaneous (s.c.) cells at the injection site, leading to inflammatory reactions and further to the formation of panniculitis.

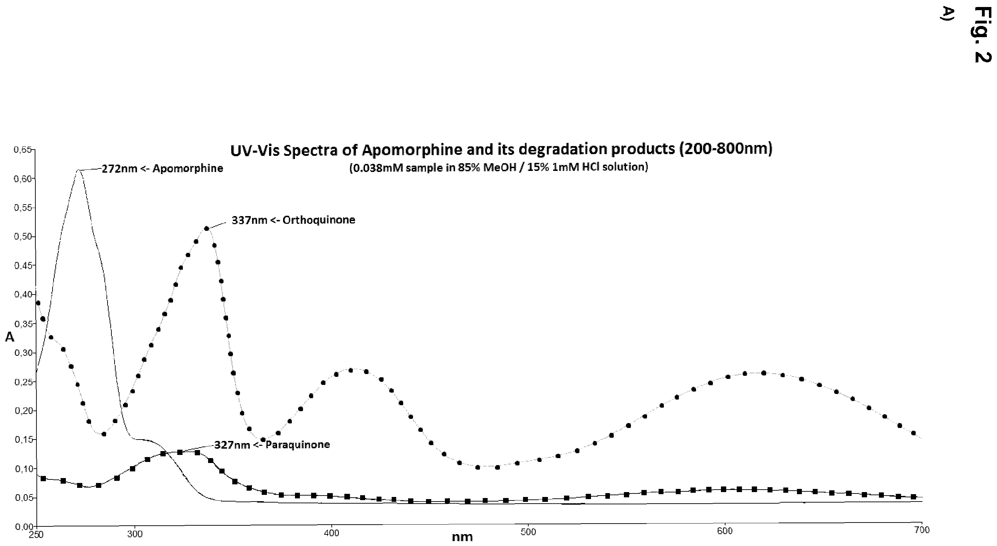

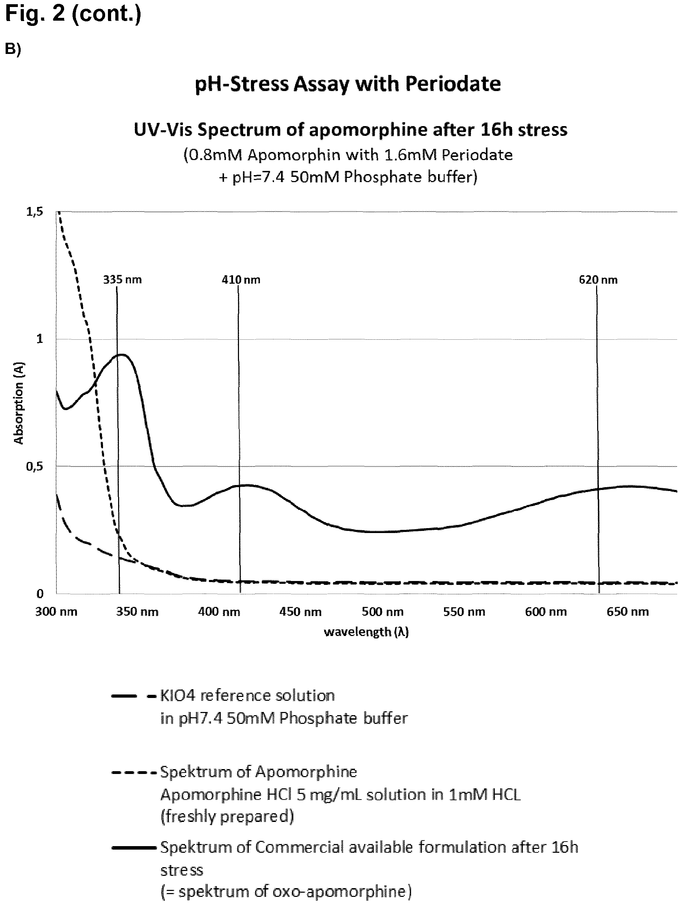

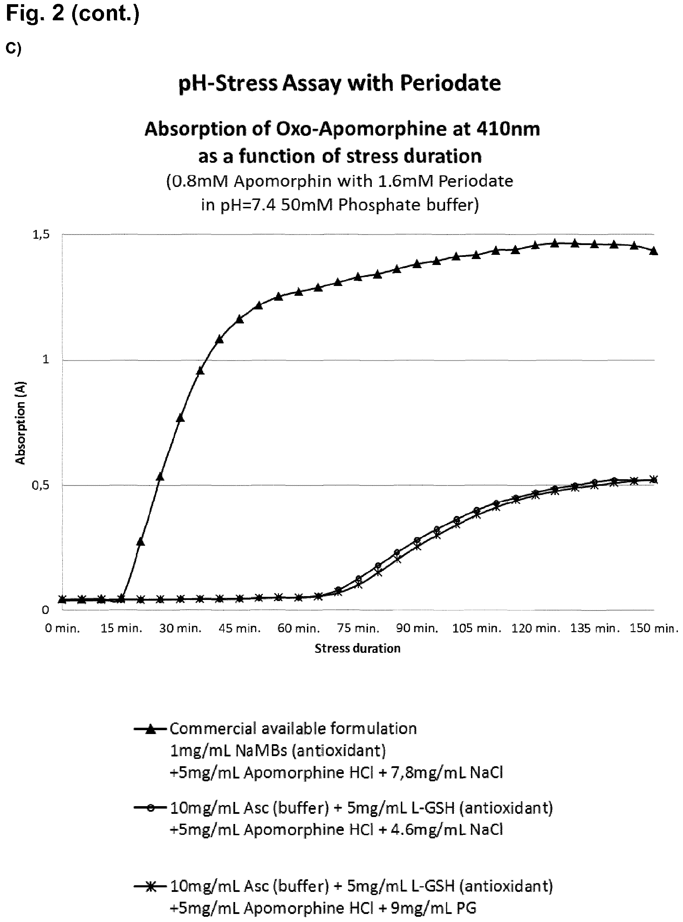

**Figure 2****: (A)** UV-Vis spectra determined for apomorphine (absorption maximum at 272 nm) and its main degradation products oxoapomorphine (ortho-quinone) (absorption maxima at 337 nm, 411 nm, and 617 nm) and apomorphine-paraquinone (absorption maxima at 327 nm and 605 nm). **(B)** UV-Vis spectra determined for potassium periodate (reference), freshly prepared apomorphine hydrochloride (reference), and a commercially available subcutaneous apomorphine hydrochloride formulation (i.e., an aqueous solution composed of 5 mg/ml apomorphine hydrochloride hemihydrate, 1 mg/ml sodium metabisulfite and 8 mg/ml sodium chloride, having a pH of 3.0 to 4.0) after 16 h oxidative stress at pH 7.4 and 1.6 mM periodate (see Example 1). Oxoapomorphine was detected in the stressed commercially available subcutaneous apomorphine hydrochloride formulation. **(C)** Formation of oxoapomorphine during pH and periodate stress in the commercially available subcutaneous apomorphine hydrochloride formulation and in exemplary aqueous compositions according to the invention (see Example 1). **(D)** pH stress assay with periodate (see Example 1).

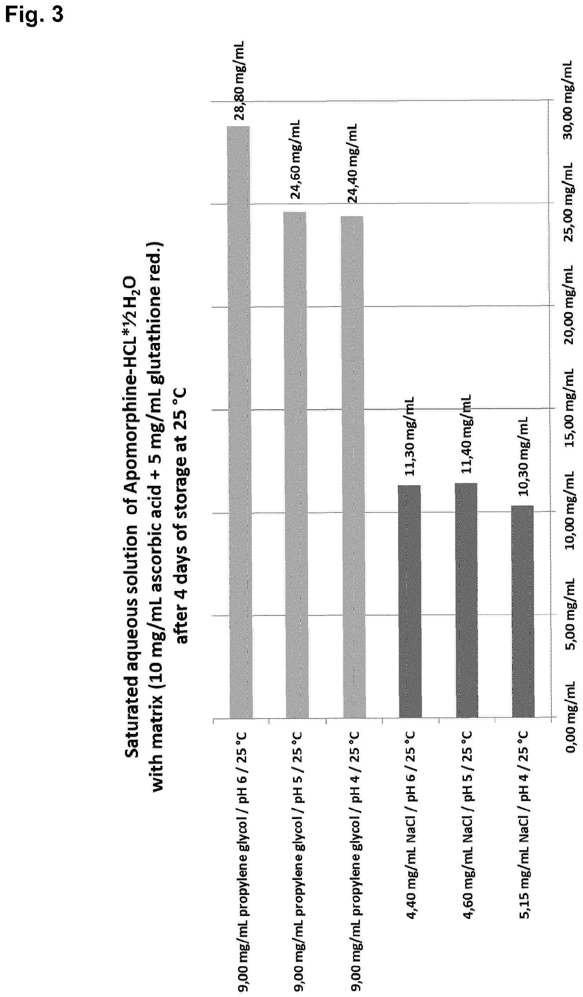

**Figure 3****:** Solubility of apomorphine hydrochloride hemihydrate in aqueous compositions according to the invention having different pH values and comprising either sodium chloride or α-propylene glycol as isotonizing agent (see Example 2).

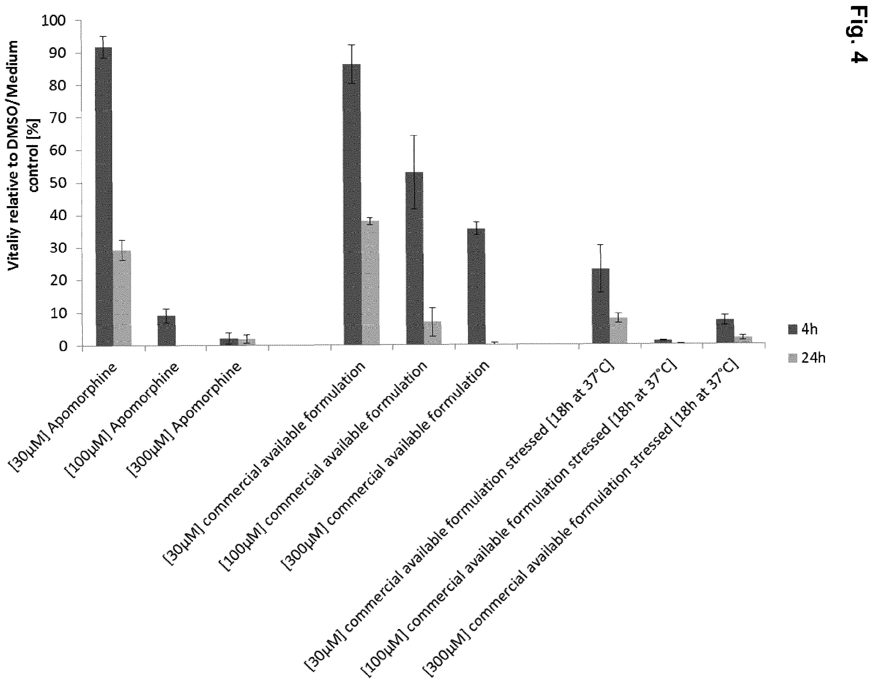

**Figure 4****:** Comparison of the cytotoxicity of apomorphine, a commercially available subcutaneous apomorphine hydrochloride formulation (i.e., an aqueous solution composed of 5 mg/ml apomorphine hydrochloride hemihydrate, 1 mg/ml sodium metabisulfite and 8 mg/ml sodium chloride, having a pH of 3.0 to 4.0) and stressed commercially available subcutaneous apomorphine hydrochloride formulation (one experiment with at least 3 different replicates). Oxidatively unprotected apomorphine showed in the resazurin viability assay a time and concentration dependent reduction of viability on cultivated NIH/3T3 cells. Current, commercially available subcutaneous apomorphine hydrochloride formulations, with the antioxidant sodium metabisulfite (i.e., aqueous solutions composed of 5 mg/ml apomorphine hydrochloride hemihydrate, 1 mg/ml sodium metabisulfite and 8 mg/ml sodium chloride, having a pH of 3.0 to 4.0) showed a slight improvement in short time incubation (4 h) but no difference in a long term incubation experiment (24 h). If the commercially available subcutaneous apomorphine hydrochloride formulation was stressed, toxic effects were even greater than with freshly prepared unprotected apomorphine. See Example 3.

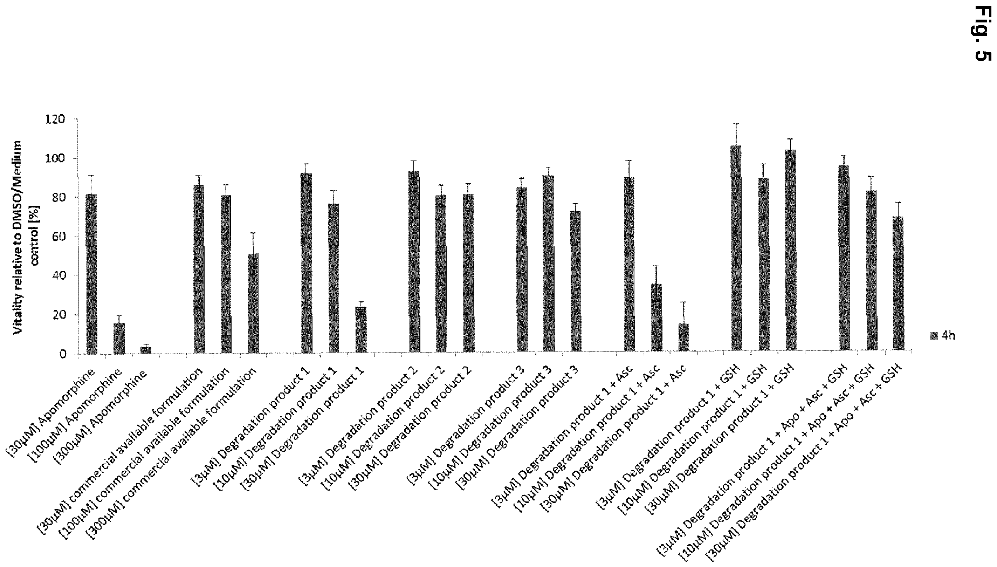

**Figure 5****:** Comparison of the cytotoxicity of apomorphine, a commercially available subcutaneous apomorphine hydrochloride formulation (i.e., an aqueous solution composed of 5 mg/ml apomorphine hydrochloride hemihydrate, 1 mg/ml sodium metabisulfite and 8 mg/ml sodium chloride, having a pH of 3.0 to 4.0) and various oxidative degradation products of apomorphine ("degradation product 1" = oxoapomorphine; 3 different experiments with at least 3 different replicates per experiment). One of 3 known synthesized oxidative degradation products of apomorphine (degradation product 1) showed a cytotoxic effect comparable to that of oxidized apomorphine on cultivated NIH/3T3 cells. Glutathione could abolish the toxic effect of oxoapomorphine. Ascorbate only prevents the oxidative degradation of apomorphine but had no beneficial effect if the oxidative degradation product oxo-apomorphine was already present in the formulation. See Example 3.

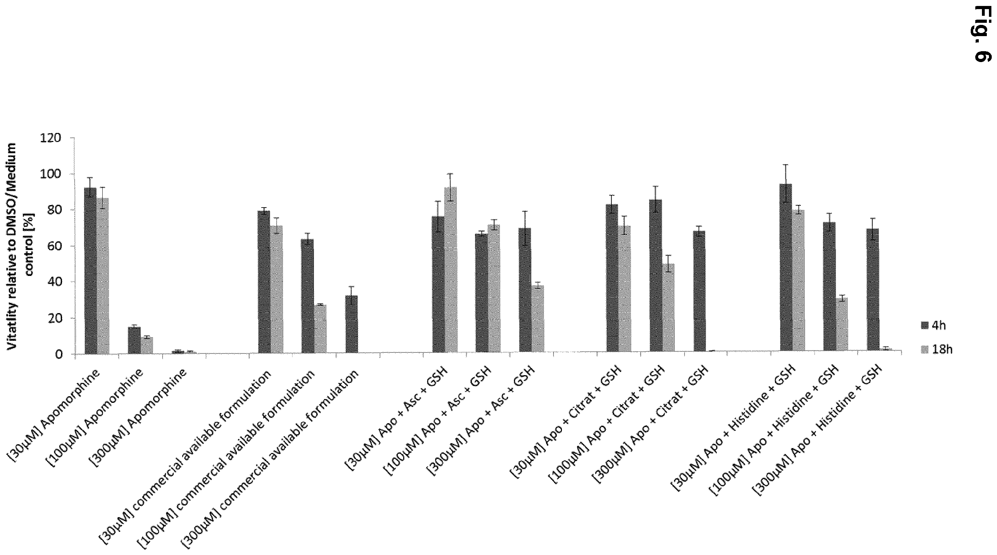

**Figure 6****:** Comparison of the cytotoxicity of apomorphine in different buffer systems (ascorbate ("Asc"), citrate, and histidine; one experiment with at least 3 different replicates). Ascorbate was used beside its antioxidative regimen as a buffering agent. The comparison of ascorbate with other buffer systems, such as citrate or histidine, which are suitable for a buffer range of pH < 6, showed that only the ascorbate buffer system was able to prevent the apomorphine-induced cytotoxicity. It has further been shown that the aqueous composition according to the invention, comprising apomorphine, ascorbate buffer, and reduced glutathione (GSH), has an advantageously reduced cytotoxic effect on the fibroblasts as compared to a commercially available subcutaneous apomorphine hydrochloride formulation (i.e., an aqueous solution composed of 5 mg/ml apomorphine hydrochloride hemihydrate, 1 mg/ml sodium metabisulfite and 8 mg/ml sodium chloride, having a pH of 3.0 to 4.0), which is particularly evident after 18 h incubation. See Example 3.

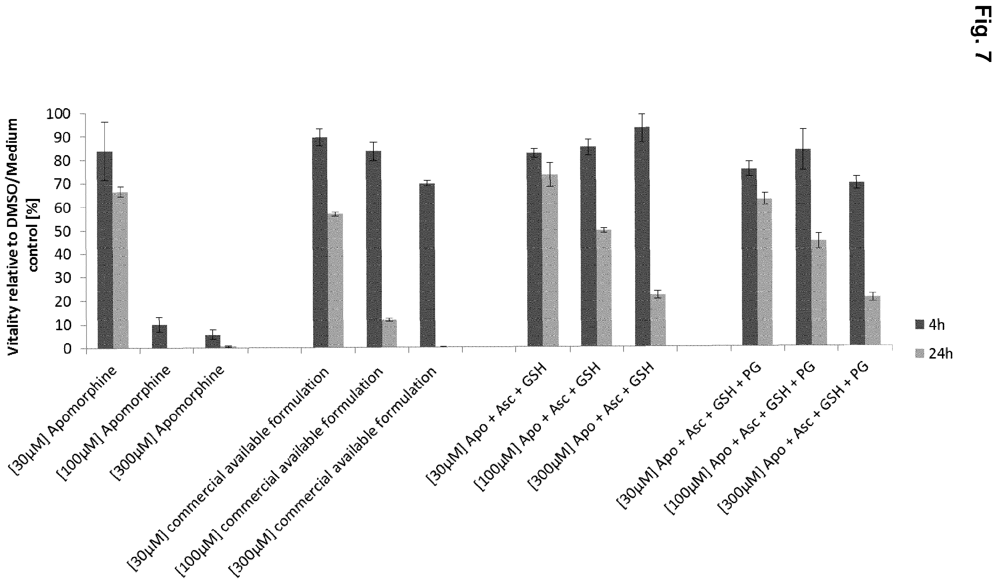

**Figure 7****:** Comparison of the cytotoxicity of different antioxidative formulations with ascorbate buffer (one experiment with at least 3 different replicates). The combination of reduced glutathione ("GSH") and ascorbate ("Asc") showed an improved cytotoxic pattern in comparison to an existing, commercially available subcutaneous apomorphine hydrochloride formulation (i.e., an aqueous solution composed of 5 mg/ml apomorphine hydrochloride hemihydrate, 1 mg/ml sodium metabisulfite and 8 mg/ml sodium chloride, having a pH of 3.0 to 4.0). α-Propylene glycol ("PG") as isotonization agent and API solubilizer did not have any influence (negative or positive) on the cytotoxic effects in the cell culture system. See Example 3.

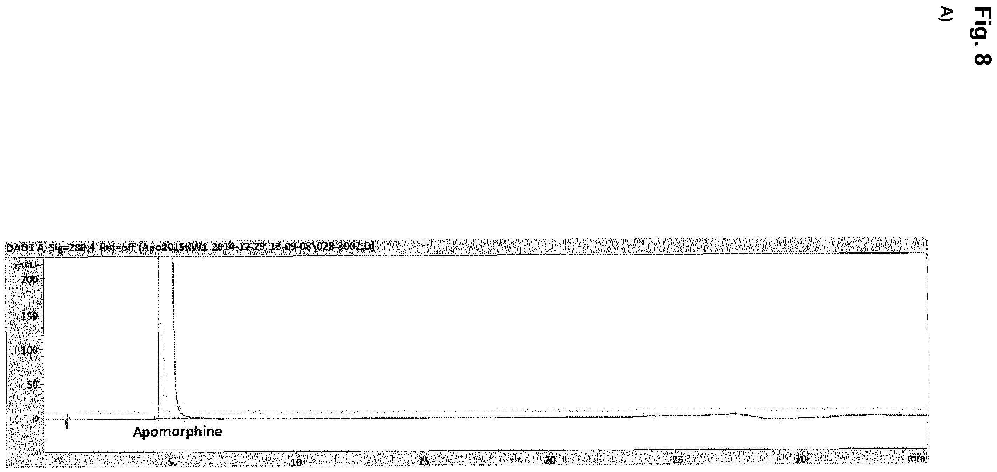

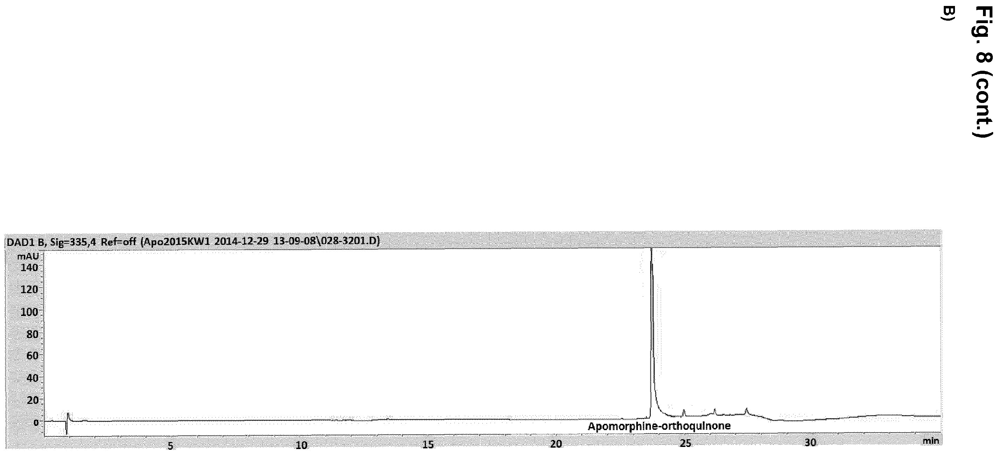

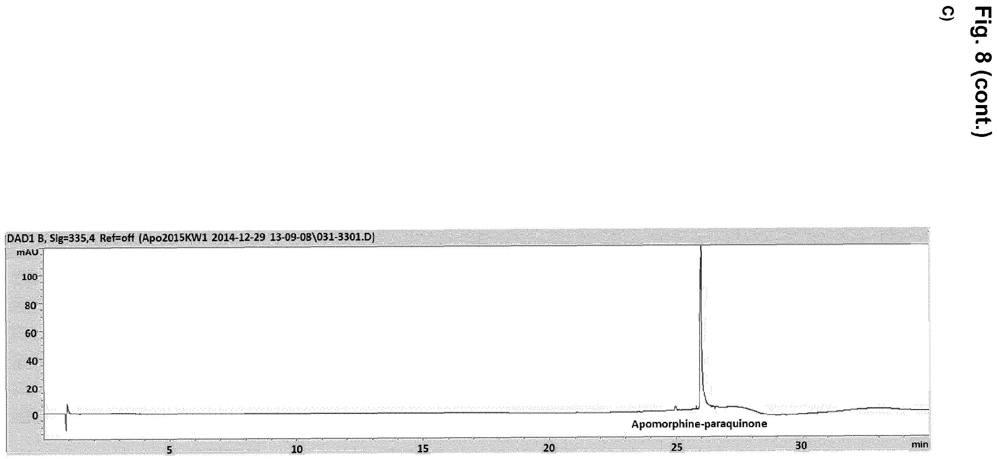

**Figure 8****:** Chromatograms of apomorphine and its degradation products oxoapomorphine and apomorphine-paraquinone (see Example 4). (A) Injection of apomorphine 5 g/L at 280 nm. (B) Injection of oxoapomorphine (= o-quinone) 1 g/L at 335 nm. (C) Injection of apomorphine-p-quinone 1 g/L at 335 nm.

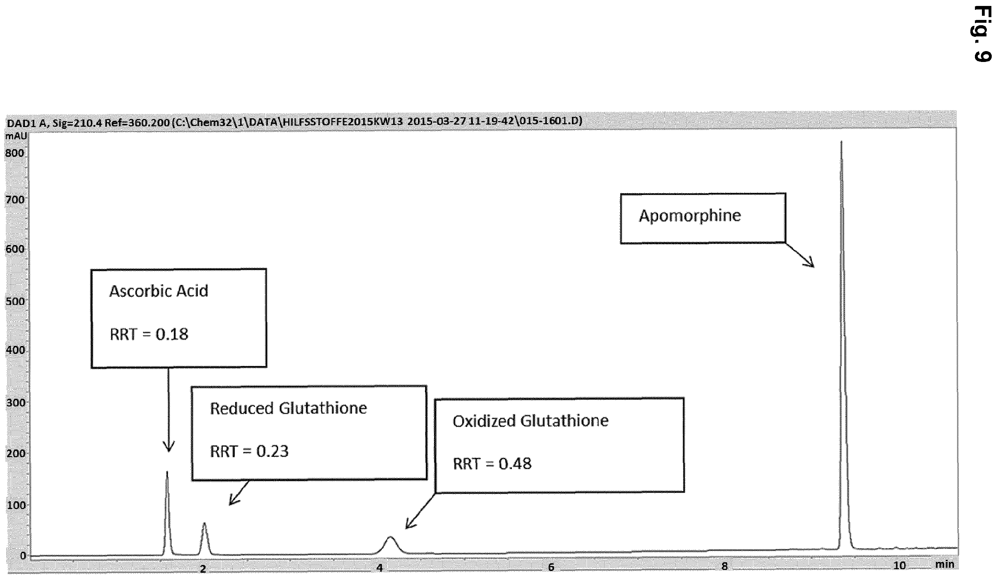

**Figure 9****:** Exemplary chromatogram of the excipient method (see Example 4). Retention times are given in relation to the apomorphine peak.

The invention will now be described by reference to the following examples which are merely illustrative and are not to be construed as a limitation of the scope of the present invention.

### EXAMPLES

### Example 1: Oxidative stress assay (pH stress and periodate)

Main target of the pH-stress assay is to obtain information about the stability against autooxidative stress conditions. The assay is based on the fact that apomorphine degrades under neutral pH conditions in a pH-dependent auto-oxidative reaction into quinone derivatives (Udvardy A, et al. Journal of molecular structure. 2011. 1002(1):37-44).

The UV-active degradation product of apomorphine (oxo-apomorphine) is used as a measuring principle for the present spectroscopic measurements. The UV-Vis spectrum of the oxo-apomorphine has three characteristic absorption maxima: 335 nm, 410 nm and 620 nm (see Figure 2A) which have been confirmed by preliminary experiments (see Figure 2B). This allows to follow the kinetics of this degradation process.

Without oxidizing agents (periodate addition), the pH-induced degradation of a commercially available subcutaneous apomorphine hydrochloride formulation (i.e., an aqueous solution composed of 5 mg/ml apomorphine hydrochloride hemihydrate, 1 mg/ml sodium metabisulfite ("NaMBs") and 8 mg/ml sodium chloride, having a pH of 3.0 to 4.0), is started only after about 30 hours, which makes it difficult to monitor formulations with higher antioxidant capacity. To accelerate the oxidative degradation of apomorphine, potassium periodate (KIO₄) was added to the solution. With this method both oxidative principles of apomorphine (the auto-oxidative, pH-dependent one and the oxidative-induced one) are covered within one assay. The results obtained in the pH stress assay with periodate are shown in Figure 2C.

The evaluation of the formulations with different antioxidant capacities is based on the time shift of the increase in absorbance values at 410 nm. This phenomenon indicates an UV-active degradation resulting from the pH-induced autoxidation and/or periodate induced oxidation of the drug measured as increase of absorbance at 410 nm. The slope of the curves corresponds to the rate of reaction. The greater the slope of a curve, the faster the UV-active degradation products are formed. The results of the new formulations were compared with the result of the commercially available subcutaneous apomorphine hydrochloride formulation (aqueous solution composed of 5 mg/ml apomorphine hydrochloride hemihydrate, 1 mg/ml sodium metabisulfite and 8 mg/ml sodium chloride, having a pH of 3.0 to 4.0) (used as control).

It has been found that the exemplary aqueous compositions according to the present invention which have been tested in this assay (comprising 5 mg/ml apomorphine HCI, 5 mg/ml reduced glutathione (L-GSH), 10 mg/ml ascorbic acid buffer, and either 4.6 mg/ml sodium chloride or 9 mg/ml α-propylene glycol) exhibit a considerably improved stability to oxidative degradation as compared to the commercially available subcutaneous apomorphine hydrochloride formulation (aqueous solution composed of 5 mg/ml apomorphine hydrochloride hemihydrate, 1 mg/ml sodium metabisulfite and 8 mg/ml sodium chloride, having a pH of 3.0 to 4.0). This is reflected, in particular, by the much longer stress duration required for absorption at 410 nm to increase (indicating the formation of the oxidative degradation product oxoapomorphine) in the case of the aqueous compositions according to the invention, and also by the much flatter slope of the absorption curve at 410 nm obtained for the aqueous compositions according to the invention, in comparison to the corresponding data obtained for the commercially available subcutaneous apomorphine hydrochloride formulation (see Figure 2C). These results demonstrate that the aqueous composition according to the invention has a highly advantageous stability to oxidative degradation. As formulation with the most effective antioxidative behavior the combination of 10 mg/ml ascorbate ("Asc") with 28 mg/ml reduced glutathione ("GSH") could be determined. Also a decrease of the slope of degradation as an indicator of the degradation velocity could be detected in the combination of ascorbic acid with GSH. This effect correlates to the amount of GSH added to the solution. This indicates the synergistic effect of the antioxidative system of ascorbic acid with reduced glutathione (see Figure 2D).

In particular, when an aqueous formulation of 5 mg/ml apomorphine HCI + 10 mg/ml GSH + 10 mg/ml Asc was subjected to the oxidative stress assay, the degradation product oxoapomorphine was found to occur after a considerably longer oxidative stress duration and at a considerably slower rate of formation, as compared to either an aqueous formulation of 5 mg/ml apomorphine HCI + 10 mg/ml GSH or an aqueous composition of 5 mg/ml apomorphine HCI + 10 mg/ml Asc, as also reflected in Figure 2D.

It has thus been demonstrated that the aqueous apomorphine formulations according to the present invention, which contain reduced glutathione in combination with ascorbic acid, exhibit a synergistically enhanced stability against oxidative degradation as compared to corresponding formulations that contain only reduced glutathione or only ascorbic acid.

### Example 2: Solubility tests

The solubility of apomorphine hydrochloride hemihydrate in aqueous compositions according to the invention, having different pH values and comprising either sodium chloride or α-propylene glycol as isotonizing agent, has been tested. The results obtained in these tests are shown in Figure 3 and in the following Table 1:

**Table 1: Solubility test results**

| **Sample** | **API** | **Matrix** | | **pH** | **concentration of saturated apomorphine-HCI solutions** |
|---|---|---|---|---|---|
| **1.** | | 10 mg/mL ascorbic acid | 5.15 mg/mL sodium chloride | 4.0 | 10.30 mg/mL |
| **2.** | | | 4.60 mg/mL sodium chloride | 5.0 | 11.40 mg/mL |
| **3.** | **apomorphine HCl x ½ H₂O** | | 4.40 mg/mL sodium chloride | 6.0 | 11.30 mg/mL |
| | | 5 mg/mL glutathione red. | | | |
| **4.** | | | 9.00 mg/mL α-propylene glycol | 4.0 | 24.40 mg/mL |
| **5.** | | | | 5.0 | 24.60 mg/mL |
| **6.** | | | | 6.0 | 28.80 mg/mL |

It has been found that α-propylene glycol (used as isotonization agent) increases the solubility of apomorphine in aqueous solution more than twice as effectively as NaCl (used as isotonization agent).

### Example 3: Resazurin-fibroblast-assay for assessment of cytotoxicity

For the assessment of cytotoxicity of the aqueous compositions of apomorphine according to the present invention in comparison to an existing, commercially available subcutaneous apomorphine hydrochloride formulation (i.e., an aqueous solution composed of 5 mg/ml apomorphine hydrochloride hemihydrate, 1 mg/ml sodium metabisulfite and 8 mg/ml sodium chloride, having a pH of 3.0 to 4.0), cultured NIH/3T3 murine fibroblasts were treated with the respective formulations.

The vitality of the fibroblasts was detected with the Resazurin Reagent (Resazurin sodium salt powder (Sigma Aldrich R7017-1G)). Vital and metabolically active cells reduce the non-fluorescent dye resazurin to the fluorescent dye resorufin by using NADH. The amount of fluorescence is proportional to the amount of vital cells. The fluorescence intensity at 535 nm (excitation) / 595 nm (emission) is detected with a plate reader.

Cells were cultured in a culture medium (DMEM high glucose) containing 10% FBS, 2 mM L-glutamine and 100 µg/ml penicilline/streptomycin under standard cell culture conditions (37°C, 5% CO₂).

### Overview of the resazurin-fibroblast-assay:

- Day 0: NIH/3T3 murine fibroblasts were seeded in 96-well plates at a density of 7,5x10³ cells/well in standard cell culture medium as described above.
- Day 1: Fibroblasts were treated with cell culture medium (serves as control) or cell culture medium + samples, respectively. Formulations to be tested always contained 5 mg/ml apomorphine and the respective amounts of excipients. The formulations were diluted before being applied to the cells according to the dilution scheme given in Tables 2, 3 and 4 below. The cells were then incubated with the respective solutions in the incubator for 4 h, 18 h, or 24 h (depending on the assay protocol). For every time point a separate 96-well plate was prepared.
- Day 1 or 2: Readout - Determination of the fluorescence intensity (resazurin readout): For the determination of the fluorescence intensity (resazurin readout) the medium (100 µl/well) was removed and 100 µl/well resazurin was added (final concentration 0.5 mM; 2.2 ml resazurin stock solution prepared in PBS with a final concentration of 2.5 mM was diluted in 8.8 ml proliferation medium). In the wells serving for the blank control (no cells) 100 µl/well resazurin (0.5 mM) was added. The 96-well plate was then incubated for 1 h in the incubator. The fluorescence intensity was then detected at 535 nm (excitation) / 595 nm (emission) with a plate reader.

**Table 2: Preparation of apomorphine solutions (Apo: apomorphine; DF = dilution factor)**

| **Apomorphine Concentration** | **Calculation** | | **Preparation of the apomorphine solution** |
|---|---|---|---|
| Apo 300mM | | | → 93,8mg in 1 ml DMSO |
| Apo 100mM | 300mM/100mM = 3 | 100 µl/3 = 33µl | → 33µl Apo [300mM Stock] + 67µl DMSO |
| Apo 30mM | 300mM/30mM = 10 | 100µl/10 = 30 µl | → 30µl Apo [300mM Stock] + 270µl DMSO |
| Apo 300µM | 300mM/300µM = 1000 | 4000µl/1000 = 4 µl | → 4µl Apo [300mM Stock] + 3996µl medium |
| Apo 100µM | 100mM/100µM = 1000 | 4000µl/1000 = 4 µl | → 4µl Apo [100mM Stock] + 3996µl medium |
| Apo 30µM | 30mM/30µM = 1000 | 4000µl/1000 = 4 µl | → 4µl Apo [30mM Stock] + 3996µl medium |
| DMSO Control | | | → 4µl DMSO + 3996µl medium |

**Table 3: Preparation of the sample solutions with a commercially available subcutaneous apomorphine hydrochloride formulation (i.e., an aqueous solution composed of 5 mg/ml apomorphine hydrochloride hemihydrate, 1 mg/ml sodium metabisulfite and 8 mg/ml sodium chloride, having a pH of 3.0 to 4.0), placebo formulation, formulation with apomorphine (note that every formulation with apomorphine contains 5 mg/ml apomorphine)**

| **Formulation** | **Calculation** | | **Preparation of the sample solution** |
|---|---|---|---|
| [900µM] formulation | 16mM Apo/900µM Apo = 17,78 | 4000µl/17,78 = 225µl | → 225 µl formulation + 3775 µl medium |
| [600µM] formulation | 16mM Apo/600µM Apo = 26,67 | 4000µl/26,67 = 150µl | → 150µl formulation + 3850µl medium |
| [300µM] formulation | 16mM Apo/300µM Apo = 53,33 | 4000µl/53,33 = 75µl | → 75µl formulation + 3925µl medium |
| [100µM] formulation | 16mM Apo/100µM Apo = 160 | 4000µl/160 = 25µl | → 25µl formulation + 3975µl medium |
| [30µM] formulation | 16mM Apo/30µM Apo = 533,33 | 4000µl/533,33 = 7,5µl | → 7,5µl formulation + 3992,5µl medium |

**Table 4: Preparation of the degradation product solutions (apomorphine to degradation product is used in a ratio of 10:1)**

| **Degradation Product Concentration** | **Calculation** | | **Preparation of the degradation product solution** |
|---|---|---|---|
| Degradation product 1: 30mM | | | → 7,89mg in 1ml DMSO |
| Degradation product 2: 30mM | | | → 7,95mg in 1ml DMSO |
| Degradation product 3: 30mM | | | → 6,39mg in 1 ml DMSO |
| Degradation product 10mM | 30mM/10mM = 3 | 100µl/3 = 33µl | → 33µl Degradation product [30mM Stock] + 67µl DMSO |
| Degradation product 3mM | 30mM/3mM = 10 | 100µl/10 = 30µl | → 30µl Degradation product [30mM Stock] + 270µl DMSO |
| Degradation product 30µM | 30mM/30µM = 1000 | 4000µl/1000 = 4µl | → 4µl Degradation product [30mM Stock] + 3996µl medium |
| Degradation product 10µM | 10mM/10µM = 1000 | 4000µl/1000 = 4µl | → 4µl Degradation product [10mM Stock] + 3996µl medium |
| Degradation product 3 µM | 3mM/3µM = 1000 | 4000µl/1000 = 4µl | → 4µl Degradation product [3mM Stock] + 3996µl medium |
| DMSO Control | | | → 4µl DMSO + 3996µl medium |

### Data analysis:

- The fluorescence intensity values [535 nm/595 nm] were determined by the mean of at least three sample replicates (blank corrected).
- The blank value (BLANK no cells) was the mean of a least three blank control values.
- For the blank correction the mean blank control values were subtracted from the sample values.
- For the determination of the vitality values relative to medium control or DMSO control, respectively, the blank-corrected sample values are divided by the mean blank corrected medium control / mean DMSO control value.

### Results:

The results obtained in the resazurin-fibroblast-assay are shown in Figures 4 to 7. In particular, oxidatively unprotected apomorphine showed a time and concentration dependent reduction of viability on cultivated NIH/3T3 cells. Current, commercially available subcutaneous apomorphine hydrochloride formulations with the antioxidant sodium metabisulfite ( aqueous solutions composed of 5 mg/ml apomorphine hydrochloride hemihydrate, 1 mg/ml sodium metabisulfite and 8 mg/ml sodium chloride, having a pH of 3.0 to 4.0) showed a slight improvement in short time incubation (4 h) but no difference in a long term incubation experiment (24 h). If the commercially available subcutaneous apomorphine hydrochloride formulation was stressed, toxic effects were even greater than with freshly prepared unprotected apomorphine (see Figure 4).

Moreover, known synthesized oxidative degradation products of apomorphine were found to have a negative effect on the viability of cultivated NIH/3T3 cells. One of these products, oxoapomorphine (referred to as "degradation product 1") showed a cytotoxic effect comparable to that of oxidized apomorphine. Glutathione could abolish the toxic effect of oxoapomorphine. Ascorbate only prevents the oxidative degradation of apomorphine but had no beneficial effect if the oxidative degradation product oxo-apomorphine was already present in the formulation (see Figure 5).

In the experiment shown in Figure 6, apomorphine was tested in different buffer systems. Ascorbate was used beside its antioxidative regimen as a buffering agent. The comparison of ascorbate with other buffer systems, such as citrate or histidine, which are suitable for a buffer range of pH < 6, showed that only the ascorbate buffer system was able to prevent the apomorphine-induced cytotoxicity.

Furthermore, it has been demonstrated in this experiment that the aqueous composition according to the invention (comprising apomorphine, ascorbate buffer, and reduced glutathione) has an advantageously reduced cytotoxic effect on the fibroblasts as compared to the commercially available subcutaneous apomorphine hydrochloride formulation (aqueous solution composed of 5 mg/ml apomorphine hydrochloride hemihydrate, 1 mg/ml sodium metabisulfite and 8 mg/ml sodium chloride, having a pH of 3.0 to 4.0), which is particularly evident after 18 h incubation (see Figure 6).

As also shown in Figure 7, the combination of glutathione (GSH) and ascorbate showed an improved cytotoxic pattern in comparison to the existing, commercially available subcutaneous apomorphine hydrochloride formulation (aqueous solution composed of 5 mg/ml apomorphine hydrochloride hemihydrate, 1 mg/ml sodium metabisulfite and 8 mg/ml sodium chloride, having a pH of 3.0 to 4.0). α-Propylene glycol as isotonization agent and API solubilizer did not have any influence (negative or positive) on the cytotoxic effects in the cell culture system.

### Example 4: Analytical procedures for the determination of apomorphine and its impurities together with ascorbic acid and glutathione (determination of stability of formulation)

### Method description:

Two different analytical methods where used. The first method is used for determination of apomorphine and apomorphine impurities, whereas the second method was used for assay ascorbic acid and glutathione in reduced and oxidized form. Method description of the apomorphine assay and impurity method is listed in Table 5 below.

**Table 5: Method description of the apomorphine method**

| **Parameter** | | | | | | | | |
|---|---|---|---|---|---|---|---|---|
| Column | WATERS Symmetry^{®} C18 3.5 µm 2.1 x 150 mm | | | | | | | |
| Detection wavelength | 280 nm for apomorphine and impurities | | | | | | | |
| | 335 nm for oxoapomorphine | | | | | | | |
| Injection volume | 10.0 µL | | | | | | | |
| Mobile Phase | A: 20 mM ammonium formate pH 2.7; | | | | | | | |
| | B: methanol | | | | | | | |
| Gradient | t [min] | 0 | 10 | 20 | 20 | 25 | 30 | 35 |
| | B [%] | 15 | 30 | 50 | 50 | 90 | 15 | 15 |
| Temperature | 40 °C | | | | | | | |
| Flow rate | 0.4 mL/min | | | | | | | |

Two main degradation products were isolated, characterized and synthesized. It is on the one hand 6-methyl-5,6-dihydro-4H-dibenzo-quinoline-10,11-dione (also referred to as oxoapomorphine or apomorphine-orthoquinone) and on the other hand 6-methyl-10-hydroxy-5,6-dihydro-4H-dibenzo-quinoline-8,11-dione (also referred to as apomorphine-paraquinone).

Oxoapomorphine is formed in an autooxidative pathway of apomorphine under acidic conditions, whereas the para-quinone results as degradation product under alkaline conditions. Oxoapomorphine and apomorphine-paraquinone are not detectable at a wavelength of 280 nm. Therefore detection wavelength for these degradation products is set to 335 nm. Retention times and detection wavelengths are listed in the following Table 6, together with chromatograms of the substances (see Figures 8A to 8C).

**Table 6: Retention times and detection wavelengths of apomorphine and its oxidative degradation products apomorphine-orthoquinone ("oxoapomorphine") and apomorphine-paraquinone**

| **Analyte** | **~ Retention time [min]** | **Detection wavelength** | **RRT** |
|---|---|---|---|
| Apomorphine | 4.62 | 280 nm | - |
| o-Quinone | 23.73 | 335 nm | 5.14 |
| p-Quinone | 25.97 | 335 nm | 5.62 |

A second method was used for determination of excipient profiles. This method can also be used for determination of assay apomorphine. Method description is shown in Table 7:

**Table 7: Method description of the excipient method**

| **Parameter** | | | | | | | | |
|---|---|---|---|---|---|---|---|---|
| Column | Synergi Hydro-RP 4.6 x 150 mm; 4 µm; Phenomenex | | | | | | | |
| Detection wavelength | 210 nm | | | | | | | |
| injection volume | 3.0 µL | | | | | | | |
| Mobile Phase | A: 0.025 M KH₂PO₄; pH 2.7 adjusted with 85% H₃PO₄; premixed with 1.6% acetonitrile | | | | | | | |
| | B: Acetonitrile | | | | | | | |
| Gradient | t [min] | 0 | 3 | 11 | 12 | 14.5 | 15 | 17.5 |
| | B [%] | 0 | 0 | 32 | 80 | 80 | 0 | 0 |
| Temperature | 20 °C | | | | | | | |
| Flow rate | 1.5 mL/min | | | | | | | |

All quality parameters of the methods were determined to be within the following limits:

**Table 8: Quality parameters**

| **Parameter** | **Limit** |
|---|---|
| Recovery | 97.5-102.5 % |
| Reproducibility | RSD < 1 |
| Precision | 97.5-102.5 % |
| Linearity | R² > 0.999 |

An exemplary chromatogram of the excipient method is shown in Figure 9.

### Results of stability study:

Analytical, chromatographic evaluation of different apomorphine formulations were evaluated in two short time stability studies:
Study 1 compared buffer system and different composition possibilities in accordance with the present invention in a 2-3 month stability study with 25°C, 40°C and 60°C conditions in comparison to a formulation which is registered on the market in the EU. All results are shown in Tables 9 to 11. Out of analytical aspects formulations of 1 to 5 mg/ml glutathione and 10 mg/ml ascorbic acid are optimal.

**Table 9: Comparative data on assay apomorphine determined by an in house HPLC method. No change in content apomorphine could be detected except in batch 085scA_18C4, where a decrease of approximately 10% in accelerated 60°C conditions could be observed.**

| | Amount of Excipient in Formulation mg/ml | | | | | Content Apomorphine [%] | 25°C | | 40°C | | 60°C | | |
|---|---|---|---|---|---|---|---|---|---|---|---|---|---|
| batch | Glutathione | Ascorbate | α-Propylene glycol | NaCl | pH | T =0 | 1M | 2M | 1M | 2M | 1M | 2M | 3M |
| 079scA_16C4 | 5 | 10 | - | - | 3.9 | 98.2 | 97.6 | 98.1 | 97.4 | 97.9 | 97.2 | 96.7 | 98.1 |
| 080scA_16C4 | 1 | 10 | - | 5.7 | 3.9 | 97.9 | 97.9 | 97.9 | 97.5 | 97.8 | 97.2 | 97.4 | 101 |
| 083scA_17C4 | 5 | 10 | 10 | | 3.9 | 97.7 | 97.8 | 98.9 | 98.7 | 98.6 | 98.1 | 98.4 | 101 |
| 087scA_17C4 | 5 | 10 | 10 | - | 5 | 98.6 | 98.6 | 101.2 | 100.8 | 100.5 | 100.5 | 100 | 100.1 |
| | | | | | | | | | | | | | |
| PB3716 commercially available finished product 1 mg/ml sodium metabisulfite | - | - | - | 7.8 | 3.3 | 97.6 | 98.6 | 98.9 | 96 | 96.9 | 96.1 | 96.6 | 101 |
| 085scA_18C4 10 mg/ml citrate 1 mg/ml Na metabisulfite 33 mg/ml mannitol | - | - | - | - | 3.9 | 98.6 | 98.9 | 99.6 | 96.1 | 95.7 | 91.1 | 90.3 | - |

**Table 10: Overview of impurities show a comparable (080scA_16C4 with 1 mg/ml glutathione) or even better impurity profile than commercially available apomorphine formulations. Citrate buffering showed an increase in impurity profile.**

| | Amount of Excipient in Formulation mg/ml | | | | | Impurities* [%] | | 25°C | | 40°C | | 60°C | | |
|---|---|---|---|---|---|---|---|---|---|---|---|---|---|---|
| batch | Glutathione | Ascorbate | α-Propylene glycol | NaCl | pH | | T=0 | 1 M | 2M | 1M | 2M | 1M | 2M . | 3M |
| 079scA_16C4 | 5 | 10 | - | - | 3.9 | Single | 0.15 | 0.37 | 0.34 | 0.29 | 0.27 | 0.27 | 0.46 | - |
| | | | | | | ***Sum*** | ***0.42*** | ***0.99*** | ***0.68*** | ***0.84*** | ***0.63*** | ***0.7*** | ***1.38*** | - |
| 080scA_16C4 | 1 | 10 | - | 5.7 | 3.9 | Single | 0.20 | 0.23 | 0.51 | 0.24 | 0.15 | 0.33 | 0.44 | |
| | | | | | | ***Sum*** | ***0.38*** | ***0.56*** | ***0.99*** | ***0.67*** | ***0.47*** | ***1*** | ***1.29*** | |
| 083scA_17C4 | 5 | 10 | 10 | | 3.9 | Single | 0.18 | 0.22 | 0.19 | 0.24 | 0.21 | 0.23 | 0.46 | 0.73 |
| | | | | | | ***Sum*** | ***0.37*** | ***0.6*** | ***0.53*** | ***0,61*** | ***0,54*** | ***0.6*** | ***1.38*** | ***1.41*** |
| 087scA_17C4 | 5 | 10 | 10 | - | 5 | Single | 0.14 | 0.20 | 0.16 | 0.22 | 0.16 | 0.46 | 0.54 | 0.97 |
| | | | | | | ***Sum*** | ***0:3*** | ***0.32*** | ***0.3*** | ***0.29*** | ***0.32*** | ***1*** | ***2.46*** | ***2.08*** |
| PB3716 | - | - | - | 7.8 | 3.3 | Single | 1.00 | 0.96 | 0.26 | 0.86 | 0.26 | 1.05 | 0.44 | 0.49 |
| commercially available finished product 1 mg/ml sodium metabisulfite | | | | | | ***Sum*** | ***1.15*** | ***1.23*** | ***0.81*** | ***1.57*** | ***1.02*** | ***1.8*** | ***1.14*** | ***1.08*** |
| 085scA_18C4 10 mg/ml citrate 1 mg/ml Na metabisulfite 33 mg/ml mannitol | - | - | - | - | 3.9 | Single | 0.59 | 1.00 | 0.30 | 3.19 | 3.62 | 8.19 | 9.02 | - |
| | | | | | | ***Sum*** | ***0.6*** | ***1.27*** | ***0.51*** | ***3.61*** | ***4.44*** | ***9.1*** | ***9.84*** | - |

| | | | | | | | | | | | | | | |
|---|---|---|---|---|---|---|---|---|---|---|---|---|---|---|
| * shown data are placebo corrected | | | | | | | | | | | | | | |

**Table 11: Assay of ascorbate in different formulations. With respect to the degradation of ascorbic acid, pH 5 is a more stable pattern than formulations at pH 3.9.**

| | Amount of Excipient in Formulation mg/ml | | | | | Content Ascorbate [%] | 25 °C | | 40°C | | 60°C | | |
|---|---|---|---|---|---|---|---|---|---|---|---|---|---|
| batch | Glutathione | Ascorbate | α-Propylene glycol | NaCl | pH | T=0 | 1M | 2M | 1M | 2M | 1M | 2M | 3M |
| 079scA_16C4 | 5 | 10 | - | - | 3.9 | 100% | 92% | 90% | 90% | 87% | 67% | 47% | n.a. |
| 080scA_16C4 | 1 | 10 | - | 5.7 | 3.9 | 100% | 85% | 86% | 79% | 82% | 46% | 31.4 | 31% |
| 083scA_17C4 | 5 | 10 | 10 | | 3.9 | 100% | 93% | 91% | 91% | 87% | 69% | 52% | n.a. |
| 087scA_17C4 | 5 | 10 | 10 | - | 5 | 100% | 97% | 97% | 97% | 94% | 88% | 82% | n.a. |
| PB3716 | - | - | - | 7.8 | 3.3 | - | - | - | - | - | - | - | - |
| commercially available finished product 1 mg/ml sodium metabisulfite | | | | | | | | | | | | | |
| 085scA_18C4 10 mg/ml citrate 1 mg/ml Na metabisulfite 33 mg/ml mannitol | - | - | - | - | 3.9 | - | - | - | - | - | - | - | - |

Study 2 based on results of study 1. Improved formulations isotonized with sodium chloride versus α-propylene glycol were stored and compared on two conditions (25°C, 60% r.h., 40°C 75% r.h.). In this study analytical methods where extended to more detailed determinations of antioxidative substances glutathione and ascorbic acid. In addition adjustment of pH was determined. Composition of batches is listed in Table 12.

**Table 12: Composition of formulations of batches manufactured for Study 2.**

| **Composition:** | **Batch No:** | | | | |
|---|---|---|---|---|---|
| | **102scA_2735** | **098scA_2535** | **099scA_2535** | **100scA_2635** | **101scA_2635** |
| **Apomorphine HCl x ½H₂O** | 5.00 mg/mL | 5.00 mg/mL | 5.00 mg/mL | 5.00 mg/mL | 5.00 mg/mL |
| **Ascorbic acid** | 10.00 mg/mL | 10.00 mg/mL | 10.00 mg/mL | 10.00 mg/mL | 10.00 mg/mL |
| **Red. glutathione** | 5.00 mg/mL | 5.00 mg/mL | 5.00 mg/mL | 5.00 mg/mL | 5.00 mg/mL |
| **NaCl** | 5.15 mg/mL | 4.60 mg/mL | 4.40 mg/mL | - | - |
| **α-Propylene glycol** | - | - | - | 9.00 mg/mL | 9.00 mg/mL |
| **pH:** | **4** | **5** | **6** | **5** | **6** |

One month results are listed in Table 13 below. Formulations with best stability results regarding assay of excipients are formulations within a pH range of 5-6. Assay and impurity profile of apomorphine is unchanged.

| **Samples** | | **1 Month 25°C/60 % r.h.** | | | | | | | |
|---|---|---|---|---|---|---|---|---|---|
| | | Visual examination | Color | pH value | Assay Apomorphine | | Assay Excipients | | |
| Batch No. | Storage | | | | Content Apo [%] | Impurities [%] | Assay ascorbic acid | Assay reduced glutathione | Assay oxidized glutathione |
| **102sc A_27 35/A** | **upside** | clear, colorless solution, free of particles | Auto B9 | 4.098 | 98.93 | 0.08 | 98.4 | 61.21 | |
| | **reverse** | clear, colorless solution, free of particles | Auto B9 | 4.095 | 99.11 | 0.07 | 98.3 | 62.81 | |
| **098sc A_25 35/A** | **upside** | clear, colorless solution, free of particles | Auto B9 | 5.136 | 100.43 | 0.07 | 100.1 | 73.04 | |
| | **reverse** | clear, colorless solution, free of particles | Auto B9 | 5.141 | 100.61 | 0.07 | 99.64 | 61.87 | 21.96 |
| **099sc A_25 35/A** | **upside** | clear, colorless solution, free of particles | Auto B9 | 5.956 | 100.11 | 0.08 | 100.65 | 73.13 | 15.91 |
| | **reverse** | clear, colorless solution, free of particles | Auto B9 | 5.948 | 100.26 | 0.06 | 100.53 | 73.12 | 15.1 |
| **100sc A_26 35/A** | **upside** | clear, colorless solution, free of particles | Auto B9 | 5.156 | 100.06 | 0.08 | 100.17 | 71.76 | |
| | **reverse** | clear, colorless solution, free of particles | Auto B9 | 5.147 | 100.16 | 0.06 | 99.99 | 72.72 | |
| **101sc A_26 35/A** | **upside** | clear, colorless solution, free of particles | Auto B9 | 6.012 | 99.74 | 0.07 | 100.22 | 71.26 | 16.6 |
| | **reverse** | clear, colorless solution, free of particles | Auto B9 | 6.017 | 99.69 | 0.07 | 99.95 | 71.64 | 16.15 |

**Table 13: Analytical results of stability study 2 of different formulations, stored in vials in two directions.**

| **Samples** | | **1 Month 40°C/75% r.h.** | | | | | | | |
|---|---|---|---|---|---|---|---|---|---|
| | | Visual examination | Color | pH value | Assay Apomorphine | | Assay Excipients | | |
| Batch No. | Storage | | | | Content Apo [%] | Impurities [%] | Assay ascorbic acid | Assay reduced glutathione | Assay oxidized glutathione |
| **102sc A_27 35/A** | **upside** | clear, colorless solution, free of particles | Auto G7 B=B8 | 4.098 | 99.49 | 0.07 | 94.16 | 21.87 | |
| | **reverse** | clear, colorless solution, free of particles | Auto BG7 Auto=B8 | 4.09 | 99.37 | 0.07 | 93.21 | 21.43 | |
| **098sc A_25 35/A** | **upside** | clear, colorless solution, free of particles | Auto B9 | 5.149 | 100.27 | 0.06 | 98.77 | 43.75 | |
| | **reverse** | clear, colorless solution, free of particles | Auto B9 | 5.151 | 100.47 | 0.07 | 98.97 | 43.9 | |
| **099sc A_25 35/A** | **upside** | clear, colorless solution, free of particles | Auto B9 | 5.886 | 99.75 | 0.07 | 100.17 | 49.44 | |
| | **reverse** | clear, colorless solution, free of particles | Auto B9 | 5.882 | 100.21 | 0.07 | 99.99 | 49.76 | |
| **100sc A_26 35/A** | **upside** | clear, colorless solution, free of particles | Auto B9 | 5.166 | 100 | 0.07 | 98.45 | 42.98 | |
| | **reverse** | clear, colorless solution, free of particles | Auto B9 | 5.151 | 99.9 | 0.07 | 99.02 | 42.65 | |
| **101sc A_26 35/A** | **upside** | clear, colorless solution, free of particles | Auto B9 | 5.926 | 99.91 | 0.07 | 99.87 | 48.73 | |
| | **reverse** | clear, colorless solution, free of particles | Auto B9 | 5.934 | 99.57 | 0.07 | 99.47 | 49.06 | |

## Claims

1. An aqueous composition comprising:
apomorphine or a pharmaceutically acceptable salt or solvate thereof;
reduced glutathione (GSH) or a pharmaceutically acceptable salt thereof; and
ascorbic acid or a pharmaceutically acceptable salt thereof;
wherein the composition has a pH of 3 to 7.4.

2. The aqueous composition of claim 1, wherein the composition comprises apomorphine hydrochloride hemihydrate.

3. The aqueous composition of claim 1 or 2, wherein the composition comprises said apomorphine or the pharmaceutically acceptable salt or solvate thereof in an amount of 5 mg/ml.

4. The aqueous composition of any one of claims 1 to 3, wherein the composition has a pH of 5 to 6.

5. The aqueous composition of any one of claims 1 to 3, wherein the composition comprises an ascorbic acid/ascorbate buffer, and wherein the composition has a pH of 5.5.

6. The aqueous composition of any one of claims 1 to 5, wherein the composition further comprises α-propylene glycol.

7. The aqueous composition of any one of claims 1 to 6, wherein the composition has an osmolality of 280 mOsm/kg to 305 mOsm/kg.

8. The aqueous composition of any one of claims 1 to 7 for use as a medicament.

9. The aqueous composition of any one of claims 1 to 7 for use in the treatment or prevention of a neurodegenerative disease/disorder.

10. The aqueous composition for use according to claim 9, wherein said neurodegenerative disease/disorder is selected from Parkinson's disease, Alzheimer's disease, Huntington's disease, neuroleptic malignant syndrome, dystonia, and schizophrenia.

11. The aqueous composition for use according to claim 10, wherein said composition is for use in the treatment or prevention of Parkinson's disease.

12. The aqueous composition of any one of claims 1 to 7 for use in the treatment or prevention of sexual dysfunction or impotence, or for use in the treatment or prevention of restless legs syndrome.

13. The aqueous composition for use according to any one of claims 9 to 12, wherein said composition is for use in preventing, reducing or ameliorating panniculitis associated with the subcutaneous administration of apomorphine, and wherein the composition is to be administered subcutaneously.

14. The aqueous composition for use according to any one of claims 9 to 12, wherein said composition is for use in preventing, reducing or ameliorating the formation of subcutaneous nodules associated with the subcutaneous administration of apomorphine, and wherein the composition is to be administered subcutaneously.

15. The aqueous composition for use according to any one of claims 9 to 12, wherein said composition is for use in preventing, reducing or ameliorating inflammation and/or irritation of the skin associated with the subcutaneous administration of apomorphine, and wherein the composition is to be administered subcutaneously.

## Patentansprüche

1. Wässrige Zusammensetzung, umfassend:
Apomorphin oder ein pharmazeutisch verträgliches Salz oder Solvat davon;
reduziertes Glutathion (GSH) oder ein pharmazeutisch verträgliches Salz davon; und
Ascorbinsäure oder ein pharmazeutisch verträgliches Salz davon;
wobei die Zusammensetzung einen pH-Wert von 3 bis 7,4 aufweist.

2. Wässrige Zusammensetzung nach Anspruch 1, wobei die Zusammensetzung Apomorphinhydrochlorid-Hemihydrat umfasst.

3. Wässrige Zusammensetzung nach Anspruch 1 oder 2, wobei die Zusammensetzung das Apomorphin oder das pharmazeutisch verträgliche Salz oder Solvat davon in einer Menge von 5 mg/ml umfasst.

4. Wässrige Zusammensetzung nach einem der Ansprüche 1 bis 3, wobei die Zusammensetzung einen pH-Wert von 5 bis 6 aufweist.

5. Wässrige Zusammensetzung nach einem der Ansprüche 1 bis 3, wobei die Zusammensetzung einen Ascorbinsäure-/Ascorbat-Puffer umfasst und wobei die Zusammensetzung einen pH-Wert von 5,5 aufweist.

6. Wässrige Zusammensetzung nach einem der Ansprüche 1 bis 5, wobei die Zusammensetzung des Weiteren α-Propylenglycol umfasst.

7. Wässrige Zusammensetzung nach einem der Ansprüche 1 bis 6, wobei die Zusammensetzung eine Osmolalität von 280 mOsm/kg bis 305 mOsm/kg aufweist.

8. Wässrige Zusammensetzung nach einem der Ansprüche 1 bis 7 zur Verwendung als Medikament.

9. Wässrige Zusammensetzung nach einem der Ansprüche 1 bis 7 zur Verwendung bei der Behandlung oder Vorbeugung einer neurodegenerativen Erkrankung/Störung.

10. Wässrige Zusammensetzung zur Verwendung nach Anspruch 9, wobei die neurodegenerative Erkrankung/Störung ausgewählt ist aus Parkinson-Krankheit, Alzheimer-Krankheit, Chorea Huntington, malignem neuroleptischem Syndrom, Dystonie und Schizophrenie.

11. Wässrige Zusammensetzung zur Verwendung nach Anspruch 10, wobei die Zusammensetzung zur Verwendung bei der Behandlung oder Vorbeugung der Parkinson-Krankheit ist.

12. Wässrige Zusammensetzung nach einem der Ansprüche 1 bis 7 zur Verwendung bei der Behandlung oder Vorbeugung von sexueller Dysfunktion oder Impotenz oder zur Verwendung bei der Behandlung oder Vorbeugung des Restless-Legs-Syndroms.

13. Wässrige Zusammensetzung zur Verwendung nach einem der Ansprüche 9 bis 12, wobei die Zusammensetzung zur Verwendung bei der Vorbeugung, Verringerung oder Verbesserung einer mit der subkutanen Verabreichung von Apomorphin in Zusammenhang stehenden Pannikulitis ist, und wobei die Zusammensetzung subkutan zu verabreichen ist.

14. Wässrige Zusammensetzung zur Verwendung nach einem der Ansprüche 9 bis 12, wobei die Zusammensetzung zur Verwendung bei der Vorbeugung, Verringerung oder Verbesserung der Bildung von mit der subkutanen Verabreichung von Apomorphin in Zusammenhang stehenden subkutanen Knötchen ist, und wobei die Zusammensetzung subkutan zu verabreichen ist.

15. Wässrige Zusammensetzung zur Verwendung nach einem der Ansprüche 9 bis 12, wobei die Zusammensetzung zur Verwendung bei der Vorbeugung, Verringerung oder Verbesserung von mit der subkutanen Verabreichung von Apomorphin in Zusammenhang stehenden Entzündungen und/oder Reizungen der Haut ist, und wobei die Zusammensetzung subkutan zu verabreichen ist.

## Revendications

1. Composition aqueuse comprenant :
de l'apomorphine ou un solvate ou sel pharmaceutiquement acceptable de celle-ci ;
du glutathion (GSH) réduit ou un sel pharmaceutiquement acceptable de celui-ci ;
et
de l'acide ascorbique ou un sel pharmaceutiquement acceptable de celui-ci ;
laquelle composition a un pH de 3 à 7,4.

2. Composition aqueuse selon la revendication 1, laquelle composition comprend du chlorhydrate d'apomorphine hémihydraté.

3. Composition aqueuse selon la revendication 1 ou 2, laquelle composition comprend ladite apomorphine ou son solvate ou sel pharmaceutiquement acceptable en une quantité de 5 mg/ml.

4. Composition aqueuse selon l'une quelconque des revendications 1 à 3, laquelle composition a un pH de 5 à 6.

5. Composition aqueuse selon l'une quelconque des revendications 1 à 3, laquelle composition comprend un tampon acide ascorbique/ascorbate, et laquelle composition a un pH de 5,5.

6. Composition aqueuse selon l'une quelconque des revendications 1 à 5, laquelle composition comprend en outre un α-propylèneglycol.

7. Composition aqueuse selon l'une quelconque des revendications 1 à 6, laquelle composition a une osmolalité de 280 mOsm/kg à 305 mOsm/kg.

8. Composition aqueuse selon l'une quelconque des revendications 1 à 7, pour une utilisation en tant que médicament.

9. Composition aqueuse selon l'une quelconque des revendications 1 à 7, pour une utilisation dans le traitement ou la prévention d'une maladie/affection neurodégénérative.

10. Composition aqueuse pour une utilisation selon la revendication 9, dans laquelle ladite maladie/affection neurodégénérative est choisie parmi la maladie de Parkinson, la maladie d'Alzheimer, la maladie de Huntington, le syndrome malin des neuroleptiques, la dystonie, et la schizophrénie.

11. Composition aqueuse pour une utilisation selon la revendication 10, laquelle composition est destinée à être utilisée dans le traitement ou la prévention de la maladie de Parkinson.

12. Composition aqueuse selon l'une quelconque des revendications 1 à 7, pour une utilisation dans le traitement ou la prévention d'une impuissance ou d'un dysfonctionnement sexuel, ou pour une utilisation dans le traitement ou la prévention du syndrome des jambes sans repos.

13. Composition aqueuse pour une utilisation selon l'une quelconque des revendications 9 à 12, laquelle composition est destinée à être utilisée dans la prévention, la réduction ou l'amélioration d'une panniculite associée à l'administration sous-cutanée d'apomorphine, et laquelle composition est destinée à être administrée par voie sous-cutanée.

14. Composition aqueuse pour une utilisation selon l'une quelconque des revendications 9 à 12, laquelle composition est destinée à être utilisée dans la prévention, la réduction ou l'amélioration de la formation de nodules sous-cutanés associée à l'administration sous-cutanée d'apomorphine, et laquelle composition est destinée à être administrée par voie sous-cutanée.

15. Composition aqueuse pour une utilisation selon l'une quelconque des revendications 9 à 12, laquelle composition est destinée à être utilisée dans la prévention, la réduction ou l'amélioration d'une inflammation et/ou d'une irritation de la peau associée à l'administration sous-cutanée d'apomorphine, et laquelle composition est destinée à être administrée par voie sous-cutanée.
